# EUROPÄISCHE PATENTANMELDUNG

(11) **EP 4 342 394 A1**
(43) Veröffentlichungstag der Anmeldung: **27.03.2024**
(21) Anmeldenummer: 23199066.4
(22) Anmeldetag: 22.09.2023
(51) Int. Cl.: A61B 17/22, A61B 17/225

(54) **VORRICHTUNG UND SYSTEM ZUR BEHANDLUNG VON ALZHEIMERERKRANKUNGEN**

(30) Priorität: 23.09.2022 DE 102022003515
(71) Anmelder: MTS Medical UG, 78467 Konstanz (DE)
(72) Erfinder: Theuer, Axel Erich, 88486 Kirchberg (DE); Gekeler, Manfred W, 78464 Konstanz (DE); Hopfenzitz,Nikolaus, 78467 Konstanz (DE); Raida, Hans-Joachim, 53639 Königswinter (DE)
(74) Vertreter: Hepp Wenger Ryffel AG

(57) **Zusammenfassung**

Die Erfindung betrifft Vorrichtungen (10) für die extrakorporale Stoßwellentherapie, ein System zur Behandlung von Alzheimererkrankungen, ein Verfahren zum Bereitstellen von Einstellparametern für eine Behandlung von Alzheimererkrankungen, ein Computerprogramm und ein Computerprogrammprodukt. Eine Vorrichtung (10; 20; 80; 90) für die extrakorporale Stoßwellentherapie, insbesondere für die Behandlung von Alzheimererkrankungen, nämlich eine elektrohydraulische Stoßwellenvorrichtung umfasst mindestens einen Stoßwellengenerator (12) und mindestens einen Stoßwellenapplikator (3). Die Vorrichtung (10) weist einen Kondensator-Entladeschwingkreis auf, dessen Entladefrequenzverteilung ein Maximum besitzt, welches unter 350kHz liegt, bevorzugt unter 300 kHz, weiter bevorzugt unter 250kHz, noch weiter bevorzugt unter 200kHz, noch weiter bevorzugt unter 150kHz, noch weiter bevorzugt unter 100kHz.

## Beschreibung

Die Erfindung betrifft Vorrichtungen für die extrakorporale Stoßwellentherapie, ein System zur Behandlung von Alzheimererkrankungen, ein Verfahren zum Bereitstellen von Einstellparametern für eine Behandlung von Alzheimererkrankungen, ein Computerprogramm und ein Computerprogrammprodukt.

Die Erfindung offenbart insbesondere eine Vorrichtung und Anordnung für die extrakorporale Behandlung von Alzheimer Erkrankungen (AD, Alzheimer Desease) in alternierenden Feldern und beschreibt die Steuerung der Vorrichtung.

Dabei werden alternierende mechanische Felder, besonders Stoßwellen, eingesetzt die über niederfrequente Frequenzbänder verlustarm durch die Schädeldecke propagieren.

Die Erfindung beinhaltet auch eine Komponente für die kombinierte Behandlung in alternierenden mechanischen (AMF) und alternierenden elektrischen Felder (AEF).

Zum Stand der Technik in der extrakorporalen Stoßwellentherapie gehören leistungsstarke elektrohydraulische Stoßwellengeräte, die ursprünglich für die Nierenstein-Zertrümmerung (Lithotripsie) entwickelt wurden. Diese elektrohydraulischen Stoßwellengeneratoren besitzen einen (Stoßwellen-)Applikator, der mit einem Stecker an einem Stoßwellengerät befestigt sein kann.

Elektrohydraulische Stoßwellengeräte umfassen einen elektrohydraulische Stoßwellengenerator mit einem Entladekondensator und einen elektrohydraulischen Stoßwellenapplikator mit einem Therapiekopf.

Zwischen dem Stoßwellengenerator und dem eigentlichen Therapiekopf kann ein Kabel in Form einer schlauchförmigen Verbindung angeordnet sein, welches stromführende Leitungen, z.B. koaxiale Hochvoltkabel sowie Steuerleitungen und ggf. auch Fluidleitungen beinhalten kann.

Das Kabel kann fest verbunden mit dem Therapiekopf sei, somit zum Applikator gehören, und mit dem Stoßwellengenerator über eine lösbare Steckverbindung verbunden sein.

Bei den elektrohydraulischen Stoßwellengeräten ist in dem Therapiekopf ein flüssigkeitsgefüllter Reflektor und ein Elektrodenpaar (Innenleiter und Außenleiter mit jeweils unterschiedlichem elektrischem Potential) angeordnet. In der Flüssigkeit, z.B. Wasser, wird durch eine anliegende Hochspannung während einer Entladung an den Elektroden über einen sich ausbildenden Ionenstrom ein heißer Plasma-Kanal erzeugt, welcher zu einer explodierenden Gasblase führt, wodurch a priori in einer Stoßwelle mit sehr hoher Amplitude bzw. Intensität und extrem kurzer Anstiegszeit resultiert.

Die positive Maximal-Druckamplitude (p+) ist bei dem transienten Druckverlauf wesentlich höher als die negative, minimale Druckamplitude (p-). Durch Reflektoren kann die elektrohydraulisch erzeugte Stoßwelle auf die Behandlungszone fokussiert werden. Durch nichtlineare Effekte während der Propagation kann sich die Stoßwelle auch noch weiter aufsteilen bzw. den hohen Druckgradienten auch während der Ausbreitung beibehalten. Die Stoßwelle breitet sich mit einer Wellengeschwindigkeit aus, die zu Beginn der Propagation höher sein kann als die lokale Schallgeschwindigkeit der Flüssigkeit im Reflektor. In diesem Fall wird die Explosion auch als Detonation bezeichnet.

Bei den elektrohydraulischen Stoßwellengeräten können die folgenden zwei Arten unterschieden werden. Bei der ersten Art wird ein geladener Kondensator durch einen Schalter über die Elektroden entladen. Bei der zweiten Art wird der Elekrodenabstand so eingestellt, dass ab einer bestimmen Kondensatorspannung eine Entladung ohne Schalten möglich ist. Diese Art der Stoßwellengeräte wird als "elektrohydraulische Stoßwellengeräte mit Selbstzündung" bezeichnet. Die in dieser Patentschrift beschriebenen Technologien sind insbesondere im Bereich der elektrohydraulischen Stoßwellengeräte mit Selbstzündung verwendbar.

Bei der elektromechanischen, d.h. elektromagnetischen oder piezoelektrischen Generierung von Druckwellen breiten sich die Wellenzüge stets mit der lokalen Wellengeschwindigkeit des Mediums aus. Dies liegt daran, dass elektromagnetische oder piezoelektrische Vorrichtungen über schwingende Oberflächen bzw. Membrane mechanische Druckwellen in einem Fluid erzeugen, die sich in einem engen Behandlungsbereich (Fokusbereich) auf ihrem Weg zum Fokuspunkt, bzw. im Fokuspunkt, zu einer Stoßwelle aufsteilen und überlagern. Aufgrund der Trägheit dieser elektromechanischen Systeme lassen sich mit Ihnen - a priori - keine Stoßwellen sondern nur Druckwellen erzeugen, die mit der lokalen Schallgeschwindigkeit des Mediums propagieren. Aufgrund der vergleichsweise geringen Aktuator-Leistungsdichte werden die elektromechanischen Geräte tendenziell eher für eng lokalisierte Behandlungen verwendet, da die verwendeten elektromechanischen Druckwellenemitter (elektromagnetischer Spulen-Aktuator oder Piezo-Aktuator) lediglich in einem Fokuspunkt eine stoßwellenartige Drucküberhöhung erreichen, d.h. anfangs auf dem Weg zu dem Fokuspunkt zunächst nur Druckwellen, jedoch keine Stoßwellen vorliegen. Entsprechende Geräte werden in der Fachwelt dennoch unter dem gebräuchlichen Gattungsbegriff der "Stoßwellengeneratoren" oder "Stoßwellengeräte" subsummiert.

Daneben gibt es ballistische Druckwellengeneratoren, die im Therapiekopf ein Rohr besitzen, in dem ein elektromagnetisch oder pneumatisch beschleunigtes Projektil am Rohrende auf ein Körperteil aufgesetzt wird und die durch den Aufprall des Projektils entstehenden Druckwellen sich vom Aufsetzpunkt aus radial ausbreiten. Neben den Stoßwellengeneratoren gibt es auch Geräte, die Ultraschallwellen oder -bursts aussenden. Bei diesen Wellenformen treten gleichermaßen hohe positive und negative Druckanteile auf. Die akustische Energie, die hierdurch in das Gewebe eingebracht wird, kann somit nur zu gleichen Anteilen auf positiven und negativen Druckamplituden zurückgeführt werden und besitzt daher eine andere Qualität als die akustische Energie eines elektrohydraulischen Stoßwellengenerators, der primär über positive Druckwellenamplituden abgestrahlt wird. Aufgrund der kontinuierlichen, sinusartigen Signale und der Gefährlichkeit der negativen Druckamplituden ist beim Einsatz von Ultraschallwandlern Vorsicht geboten, wenn diese mit hoher Leistung betrieben werden. Insofern ist die akustische Energie allein kein hinreichendes Maß für die Beurteilung der Sicherheit einer Behandlung mit Stoßwellengeneratoren, da es u.a. auch auf die Höhe der lokalen Druckamplituden und das Verhältnis von positiven und negativen Druckamplituden ankommt. Zudem ist insbesondere bei einer Fokussierung lokal die Energie kein relevantes Maß, da lokal die Intensität (Energie/pro Fläche) ausschlaggebend ist.

Allen Geräten zur Erzeugung von Stoß- bzw. Druckwellen ist in der Regel gemeinsam, dass sie Applikatoren besitzen, die über ein stromführendes Hochvoltkabel (und ggf. auch noch mit Fluidleitungen) mit dem Stoßwellengenerator verbunden sind. Teil des Applikators ist der Therapiekopf, über den die Stoß- bzw. Druckwellen am Körper bzw. auch am Patientenkopf appliziert werden können.

In CN105126262B wird eine Methode und eine Vorrichtung zur ultraschallbasierten Aktivierung von Zellen offenbart. Das Verfahren umfasst die Behandlung von pathologischen Zuständen, einschließlich, aber nicht beschränkt auf Parkinson Krankheit, Alzheimer-Krankheit, Stupor, Epilepsie, Schlaganfall, Melancholie, Schizophrenie, Sucht, Schmerzen des Nervensystems, Kognitions-/Gedächtnisstörungen, Diabetes, Adipositas, Zwangsneurose, Schädel-Hirn-Trauma, posttraumatische Belastungsstörung (PTSD), Rückenmarkverletzung, peripheres Nervensystem, Migräne, Epilepsie-Krankheit des menschlichen oder tierischen Körpers.
Krankhafte Proteinablagerungen weisen spezifische physikalische Eigenschaften auf, die auf ein distinktes dynamisches Verhalten führen. Über die physikalischen Parameter werden spezifische alterierende mechanische Felder bestimmt, die zu einer Stoßwelleninduzierten Fragmentation und Auflösung von krankhaften Proteinablagerungen führen.

Die Nervenzellen des menschlichen Gehirns kommunizieren miteinander über elektrische Impulse. Damit die Nachbarzelle die Information aufnehmen kann, werden die elektrischen Impulse in chemische Botenstoffe konvertiert.

Amyloid-Plaques sind harte, unauflösliche Eiweißablagerungen, die sich aus dem Protein-Fragment "Beta-Amyloid" zusammensetzen. Dieses Protein-Fragment wird bei der Alzheimer-Krankheit nicht mehr vom Gehirn abgebaut.

Der zweite Typ von Eiweißablagerungen sind die sogenannten Tau-Fibrillen. Hierbei handelt es sich um unauflösliche, gedrehte Fasern, welche sich aus dem Tau-Protein zusammensetzen. Dieses Protein ist ein wichtiges Bauteil von Transport-Strukturen innerhalb der Nervenzellen. Bei Alzheimer Patienten behindern Tau-Proteine den Nährstofftransport.

Bei vielen Alzheimer-Betroffenen findet sich Amyloid nicht nur zwischen den Nervenzellen, sondern auch in kleineren Blutgefäßen des Gehirns. Deshalb leiden etwa 15 Prozent aller Alzheimererkrankten Menschen nicht nur an einer Demenz des Alzheimer-Typs, sondern zugleich auch an einer vaskulären Demenz. Die kleinen Blutgefäße im Gehirn sind mit Amyloid verengt, was wiederum Schlaganfälle auslösen kann.

Plaques, die bei Alzheimer auftreten, produzieren freie Radikale und sauerstoffhaltige Verbindungen.

Unter Beta-Amyloid werden Proteine verstanden, die als Hauptauslöser von Alzheimer und anderen demenziellen Veränderungen gelten. Diese Proteine kommen auch bei gesunden Menschen vor. Krankmachend sind sie, wenn sie nicht abgebaut werden können. Sie führen dann zu einer Störung neuronaler Impulse, Signale können nicht weitergegeben werden.

Die Plaques sind in Form und Größe sehr variabel; in für AD-immungefärbten Gewebeschnitten umfassen sie eine Größenverteilungskurve mit einer durchschnittlichen Plaquefläche von 400-450 µm².

Amyloid-Materialien sind sehr steif. Die physikalischen Eigenschaften von Amyloid Plaques variieren, so z.B. variert der Elastizitätsmodul zwischen 108Pa und 1010Pa. Beta-Amyloid ist das Fragment eines Proteins, das aus einem größeren Protein mit dem Namen APP (Amyloid-Vorläufer-Protein) herausgeschnitten wird. Im gesunden Gehirn werden diese Fragmente zersetzt und vernichtet. Bei der Alzheimer-Krankheit aber häufen sie sich zu harten, unauflöslichen Plaques an.

In AU2003250102B2 wird ein Verfahren beschrieben, bei dem mit über Beta-Amyloid Fragmente ein Impfwirkstoff gegen Alzheimer entwickelt wird. Unter Verwendung einer Elastographie-Bildgebungsmodalität werden die mechanischen Eigenschaften der Proteinablagerungen bestimmt.

In JP2020501734A wird Ultraschallenergie zur Behandlung von degenerativer Demenz eingesetzt. Der Fokus des Ultraschallwandlerstrahls wird auf einen Zielbereich des Gehirns gerichtet, was dazu beiträgt, Material zu entfernen, das sich im interstitiellen Weg ansammelt, der zumindest teilweise auf degenerative Demenz anspricht. In einem Beispiel kann der Zielbereich des Gehirns den Hippocampus beinhalten und die degenerative Demenz kann die Alzheimer-Krankheit sein. Ultraschallstrahlen können Gehirngewebe mit einer Frequenz stimulieren, die der natürlich vorkommenden Tiefschlaf-Burst-Frequenz von Neuronen und nachfolgenden astrozytischen Aktivierungsmustern entspricht, wodurch konvektive Prozesse als Reaktion auf die Abgabe von gelösten Stoffen im Gehirn angetrieben werden. Beispielsweise kann ein Wandler eine Burst-Frequenz von 1 bis 4 Hertz erzeugen, um die Tiefschlaf-Hirnfunktion zu stimulieren, die hilft, Amyloid-Plaque zu entfernen.

In EP2722012B1 wird eine Vorrichtung zur Behandlung des menschlichen oder tierischen Gehirns mit Stoßwellen mittels eines Stoßwellenwandlers beschrieben, die es ermöglicht, eine vorbestimmte Dosis in einen behandelten Bereich und/oder Volumen des Gehirngewebes zu applizieren, der deutlich größer ist als die Größe des Fokusflecks des Stoßwellenwandlers. Eine Energie von 0,01 bis 1 mJ/mm², am meisten bevorzugt 0,1 bis 0,3 mJ/mm², wird nach Absorptionskorrektur vorgeschlagen. Die Impulsrate liegt zwischen 1 und 20, am meisten bevorzugt 3-8 Impulsen pro Sekunde. Es muss eine Mindestdosis vorliegen, um eine therapeutische Wirkung zu erzielen, z. für Thrombolyse, erhöhte Durchblutung, Stoffwechsel, Entfernung von Amyloidbeta oder Stimulation von Nerven oder Gehirnzellen. Gemäss EP2722012B1 muss das Überschreiten einer Höchstdosis unbedingt verhindert werden, da dies zu gefährlichen Nebenwirkungen wie Blutungen führen kann.

Aufgrund der oben beschriebenen, systembedingten Fokussierung bei elektromechanischen Stoßwellengeräten zur Erreichung einer Stoßwelle wurde in der Patentschrift ein "Kartierungsgerät" (bzw. 3D-Trackingsystem) vorgestellt, um die Bewegung des Fokusflecks über mehrere Positionen zusammen mit der an jeder der Positionen applizierten Stoß-bzw. Druckwellendosis abzubilden. Mit einem solchen System wird versucht, anhand von dargestellten Patientendaten die Behandlung zu prüfen bzw. Nebenwirkungen zu vermeiden, u.a. zu gewährleisten, dass die gleichen Gehirnareale aufgrund der systembedingt fokussierten Wellenfelder nicht zu häufig bzw. zu intensiv unregelmäßig beaufschlagt werden.

Durch sehr präzise, monotone Wiederholung von Stoß- oder Druckwellen mit nahezu gleicher Amplitude, wie dies mitunter bei elektromagnetischen oder piezoelektrischen Stoßwellengeneratoren (subsummiert unter "elektromechanischen" Stoßwellengeneratoren) geschieht, ist es nicht auszuschließen, dass lokal hohe Energiekonzentrationen im Gehirn auftreten können.

Fokussierte elektromechanische Stoßwellengeräte können daher zu hohen Gewebebelastungen führen und werden vermutlich aus diesem Grund aktuell mit aufwändigen 3D-Trackingsystemen verwendet. Die Trackingsysteme können höchstens grob die Exposition bzw. lokal verabreichte Dosis bestimmen, liefern jedoch keine detaillierte Analyse der Auswirkungen auf der Gewebe- bzw. Zellebene im Gehirn. Aber gerade die genaue Berücksichtigung der lokalen physikalischen Wirkung der Stoßwellen auf den Schädel sowie die Gewebe- und Zellstruktur im Gehirn ermöglicht für die Behandlung höchstmögliche Effektivität und Sicherheit. Neben der Dichte und Elastizität des zu behandelnden Gewebes und des Schädels spielt u.a. auch der Einfallswinkel der Stoßwellen auf den Schädel eine Rolle, da sich durch den gekrümmten Schädel die Richtung der Wellenpropagation stark ändern kann. So kann es passieren, dass es auch durch unterschiedliche Winkel bei der Applizierung von fokussierten Stoß- oder Druckwellen u.U. ungewollt zu lokalen, hohen Energiekonzentrationen im Gehirn kommt.

Es wurde ebenfalls durch Messungen und Simulationen festgestellt, dass es schwierig ist, den Schädel des Menschen (höhere Dichte und Steifigkeit als das Gehirn) mit Stoßwellen zu durchdringen. Bei Exposition mit Stoßwellen, die viel Energie in hohen Frequenzbändern besitzen, kann das schädelnahe Gewebe im Gehirn geschädigt werden, unter anderem durch die mit der Frequenz zunehmende Absorption bzw. lokale Erwärmung.

In US8697627B2 wird darauf hingewiesen, dass neuroinflammatorische Prozesse auch zur Pathophysiologie der Alzheimer-Krankheit beitragen und dass Mikroglia, die residenten Entzündungszellen des Gehirns, sich in einem hochaktivierten Zustand befinden. Extrazelluläre Plaques sind Klumpen eines normalerweise harmlosen Proteins namens Beta-Amyloid (Aß), welches die Kommunikation zwischen Gehirnzellen beeinträchtigen kann.

In US8697627B2 wird ein Verfahren zur Verringerung der Amyloid-Plaque-Belastung mit Hilfe einer niedrigen Dosis eines p38-MAPK-Inhibitors dargelegt. Das Verfahren umfasst die Bildgebung des Gehirns, die Bestimmung der Anzahl der Amyloid-Plaques und die Verabreichung einer therapeutisch wirksamen Menge eines p38-Inhibitors, wenn die Anzahl der Amyloid-Plaques einen vorbestimmten Schwellenwert überschreitet.

Es ist Aufgabe der Erfindung, eine Vorrichtung, ein System, ein Verfahren und ein Computerprogramm bereit zu stellen, mit welchen die Nachteile des Bekannten überwunden werden, und insbesondere eine Anordnung für die extrakorporale Behandlung von AD und Demenz in AMF und AEF und die Steuerung der Vorrichtung zu offenbaren, durch welche eine hohe Effektivität und Sicherheit in der Behandlung erreicht wird, sodass insbesondere hohe Druckspitzen im Gehirn vermieden werden. Außerdem ist es wichtig, die Durchdringung der Schädeldecke mit einer Stoßwelle zu optimieren.

Es ist insbesondere eine weitere Aufgabe der Erfindung zu erreichen, dass krankhafte Proteinablagerungen im Gehirn fragmentiert und aufgelöst werden, anti-inflammatorische Prozesse induziert werden, die Gehirndurchblutung verbessert und die Neubildung von Blutgefäßen (Angiogenese) initiiert wird.

Lösungen des Problems sind in den unabhängigen Anspruch beschrieben. Die abhängigen Ansprüche beziehen sich auf weitere Verbesserungen der Erfindung.

Dia Aufgabe wird gelöst durch eine elektrohydraulische Vorrichtung für die extrakorporale Stoßwellentherapie, insbesondere für die Behandlung von Alzheimererkrankungen, die mindestens einen Stoßwellengenerator und mindestens einen Stoßwellenapplikator umfasst. Der Stoßwellenapplikator umfasst mindestens den eigentlichen Therapiekopf, der an dem Patienten angelegt werden kann und insbesondere ein Verbindungskabel und einen Stecker.

Die Vorrichtung weist einen Kondensator-Entladeschwingkreis auf, dessen Entladefrequenzverteilung ein Maximum besitzt, welches unter 350kHz liegt, bevorzugt unter 300 kHz, weiter bevorzugt unter 250kHz, noch weiter bevorzugt unter 200kHz, noch weiter bevorzugt unter 150kHz, noch weiter bevorzugt unter 100kHz. Die Entladefrequenzverteilung kann mehrere lokale Maxima besitzen. Mindestens eines davon, bevorzugt das Hauptmaximum, liegt im niederfrequenten Bereich.

Bei gegebenen Kennzahlen des Entladekondensators, zum Beispiel einer Kapazität zwischen 10-800 nF, insbesondere 150-250 nF, und/oder einer Entladespannung des Kondensators zwischen 1kV und 20 kV, vorzugsweise zwischen 1kV und 7.5kV, und/oder einer im Kondensator des Entladeschwingkreises gespeicherter Energie von 0.5J bis 25J, insbesondere 0.5J bis 5J, ist die Induktivität, insbesondere die Länge eines Kabels zwischen Stoßwellengenerator und Stoßwellenapplikator, so ausgelegt, dass die erfindungsgemäße Lage des Maximums der Frequenzverteilung erreicht wird.

Die Aufgabe wird weiterhin gelöst durch eine Vorrichtung für die extrakorporale Stoßwellentherapie, insbesondere wie oben beschrieben, insbesondere für die Behandlung von Alzheimererkrankungen, nämliche eine elektrohydraulische Stoßwellenvorrichtung umfassend mindestens einen Stoßwellengenerator und mindestens einen Stoßwellenapplikator. Der Stoßwellenapplikator umfasst mindestens den eigentlichen Therapiekopf, der an dem Patienten angelegt werden kann und insbesondere ein Verbindungskabel und einen Stecker.

Die Vorrichtung umfasst eine Frequenzsteuereinheit, die dazu ausgelegt ist, den Stoßwellengenerator so einzustellen, dass ein Kondensator-Entladeschwingkreis der Vorrichtung eine Entladefrequenzverteilung besitzt, deren Maximum unterhalb von 350kHz liegt, bevorzugt unter 300 kHz, weiter bevorzugt unter 250kHz, noch weiter bevorzugt unter 200kHz, noch weiter bevorzugt unter 150kHz, noch weiter bevorzugt unter 100kHz.

Eine solche Vorrichtung ist in der Lage, Stoßwellen mit Frequenzbändern abzugeben, deren dominante Maxima im Bereich zwischen 80KHz und 850kHz, bevorzugt zwischen 80kHz und 450kHz, noch bevorzugter zwischen 80Hz und 350 kHz liegen. Die Frequenzbänder beziehen sich auf Frequenzverteilung in der Fouriertransformierten des Druckverlaufs der Stoßwelle, die den Applikator verlassen hat.

Die dominanten Maxima der Stoßwellen beinhalten mindestens 50%, vorzugsweise 70% und noch vorzugsweiser 90% der Gesamtenergie der Stoßwelle.

Erfindungsgemäß werden mechanische Felder eingesetzt. Die mechanisch wirksamen, zeitveränderlichen Felder sind dabei auf die zu behandelnden Areale abgestimmt. Die mechanischen Felder, bei denen lokal Kräfte wirken, werden durch Stoßwellen hervorgerufen und propagieren durch das Gehirn. Die Felder bzw. Stoßwellenfelder zeichnen sich aus durch die Stoßwellen-Druckamplituden, die Frequenzanteile der Stoßwellen, den zeitlichen Abstand zweier oder mehrerer aufeinanderfolgender Stoßwellen (d.h. die Impulsfrequenz bzw. Impulsrate), die Wellengeschwindigkeit der Stoßwelle, die sich nach der Entstehung mit höherer Geschwindigkeit als die Schallgeschwindigkeit des übertragenden Mediums ausbreitet und durch Nichtlinearitäten Ihren Stoßwellencharakter trotz Absorption bei der Propagation beibehält, sowie lokale schwingungstechnische Eigenschaften des Schädels und des Gehirns aus (insbesondere die Dichte, Steifigkeit und Absorption) aus. Mit "lokal" sind Bereiche im Schädel bzw. Gehirn bis hin zu kleinsten Strukturen gemeint.

Es werden bevorzugt Stoßwellenfelder eingesetzt, die über niederfrequente Frequenzbänder verlustarm durch die Schädeldecke propagieren und im Gehirn gleichmäßig verteilte Druckfelder aufbringen, die zu einer selektiven Fragmentation und Auflösung von krankhaften Proteinablagerungen führen.

Durch die Applizierung der Stoßwellen über tieffrequente Frequenzbänder und durch die gleichzeitige Behandlung größerer Gehirnbereiche werden krankhafte Proteinablagerungen im Gehirn fragmentiert und aufgelöst, anti-inflammatorische Prozesse induziert, die Gehirndurchblutung verbessert und die Neubildung von Blutgefäßen initiiert.

Die Frequenzsteuereinheit kann dazu ausgelegt sein, die Lage Frequenz mindestens des Maximums mindestens eines der Frequenzbänder des Drucksignals einzustellen bzw. bevorzugt zu niedrigeren Frequenzen zu verschieben. Insbesondere kann die Einstellung über die Einstellung einer Entladefrequenz des elektrohydraulischen Stoßwellengenerators erfolgen.

Die Entladefrequenz des Entladeschwingkreises eines erfindungsgemäßen StOßwellengenerators liegt vorzugsweise unter 450kHz, noch vorzugsweiser unter 400kHz, noch vorzugsweiser unter 350kHz, noch vorzugsweiser unter 300 kHz, noch vorzugsweiser unter 250kHz, noch vorzugsweiser unter 200kHz, noch vorzugsweiser unter 150kHz, noch vorzugsweiser unter 100kHz.

Die Kapazität des Kondensators des Entladeschwingkreises eines erfindungsgemäßen Stoßwellengerätes kann bei 50-800 nF, vorzugsweise 100-400 nF, weiter bevorzugt 100-300 nF, weiter bevorzugt 150-250 nF, liegen.

Die Spannung des Kondensators eines Entladeschwingkreises bei eines erfindungsgemäßen Stoßwellengerätes kann zwischen 1kV und 20 kV, insbesondere zwischen 1kV und 10 kV, weiter insbesondere zwischen 1kV und 7.5kV liegen.

Der Entladeschwingkreis der Vorrichtung kann einen Kondensator aufweisen und die in dem Kondensator gespeicherte Energie eines erfindungsgemäßen Stoßwellengerätes bei 0.5J bis 25J liegt, insbesondere bei 0.5J bis 5J.

Die Induktivität des Entladeschwingkreis der elektrohydraulischen Stoßwellenvorrichtung kann durch eine zusätzliche Induktivität erhöht werden und damit die Entladefrequenz signifikant reduziert werden, wodurch mehr Energie der Stoßwelle in tieffrequenten Frequenzbänder propagiert und dadurch auch leichter den Schädel durchdringen kann, denn die höhere Dichte und Festigkeit des Schädels wirkt wie ein Tiefpassfilter, der bevorzugt Druckwellen unterhalb von etwa 450 kHz durchlässt und hochfrequente Druckwellen von der Propagation ins Gehirn sehr stark dämmt bzw. dämpft.

Bei den elektrohydraulischen Stoßwellengeräten kann die Entladefrequenz des Kondensators beispielsweise durch die Einbringung einer zusätzlichen Induktivität reduziert werden.

Die Frequenzsteuereinheit kann dazu ausgelegt sein, die Entladefrequenzverteilung über eine Veränderung der Induktivität des Entladekreises einzustellen, insbesondere über eine Festlegung der Länge eines Kabels zwischen Stoßwellengenerator und Stoßwellenapplikator.

Ein koaxiales Hochvoltkabel, welches den Stoßwellengenerator mit dem Applikator bzw. Therapiekopf verbindet, besitzt in etwa eine Induktivität von beispielsweise 278 nH/m. Wenn das koaxiales Hochvoltkabel z.B. 1.5m lang ist, so besitzt es eine Induktivität von ca. 417nH. Um die Entladefrequenz signifikant zu reduzieren, muss man diese Induktivität des Entladekreises massiv erhöhen, z.B. auf etwa den doppelten, vorzugsweise etwa den dreifachen, noch vorzugsweiser den etwa den vierfachen, noch vorzugsweiser etwa den fünffachen Wert dieser Induktivität oder noch vorzugsweiser auf einen Wert, der mehr als die fünffache Induktivität beträgt.

Die zusätzlichen Induktivitäten lassen sich beispielsweise durch die Verwendung langer koaxialer Hochvolt-Kabel zwischen dem Stoßwellengenerator, insbesondere dessen Hochvolt-Modul, und dem Stoßwellen-Applikator realisieren, wobei die koaxialen Hochvoltkabel länger als 1,5m bzw. vorteilhafter über 2m zum bzw. noch vorteilhafter über 3m bzw. noch vorteilhafter über 5m zum Einsatz kommen. Die koaxialen Hochvoltkabel können auch Längen von bis zu 10 Metern, 15 Metern und mehr besitzen. Die koaxialen Hochvoltkabel samt Therapiekopf können ausgetauscht werden. Die Induktivität kann zum Beispiel verändert werden, indem zwischen verschieden langen axialen Hochvoltkabeln geschaltet wird oder ein zusätzliches koaxiales Hochvoltkabel zwischen Therapiekopf und dem Stoßwellengenerator geschaltet wird. Die Schaltung sollte nicht im Betrieb erfolgen und muss hochspannungssicher erfolgen.

Die koaxialen Hochvoltkabel können auch zu einem Teil im Standgerät verbaut sein. Die Länge des eingebauten koaxialen Hochvoltkabels ist bevorzugt länger als 2m, insbesondere länger 5m und besonders bevorzugt ist das koaxiale Hochvoltkabel zwischen 9 und 20 Metern lang. Insbesondere kann die Länge des Kabels zwischen Längen von 2m bis 20m variiert werden.

Das koaxiale Hochvoltkabel kann mehrfach zwischen Applikator und dem Stecker am Stoßwellengerät hin- und herlaufen, wobei kritische Biegungen des Hochvoltkabels im Stecker bzw. im Applikator Therapiekopf untergebracht sein können.

Alternativ bzw. ergänzend kann auch eine Toroid- oder Ringkernspule im Stoßwellengerät verwendet werden, durch die der stromführende Leiter des Koaxialkabels einmal oder mehrfach hindurchgeführt wird.

Eine Spule oder mehrere Spulen mit unterschiedlichen Induktivitäten können in einer, insbesondere abgeschirmten, Kassette angeordnet sein, mit welcher ein bestehendes Stoßwellengerät nachgerüstet werden kann.

Die Frequenzsteuereinheit kann dazu ausgelegt sein, eine Spule hinzu zu schalten, eine Spule aus dem Entladekreis zu entfernen oder zwischen Spulen mit unterschiedlichen Induktivitäten hin und her zu schalten.

Alternativ oder zusätzlich können Mittel zur Erzielung einer tieferen Entladungsfrequenz eines Kondensatorschwingkreis verwendet werden, die aus dem Stand der Technik in der Elektrotechnik bekannt sind.

Die Induktivität des Entladeschwingkreis kann auch reduziert werden auf den halben, den dritten, vierten oder fünften Teil bzw. einen noch kleineren Wert, um die Entladefrequenzen entsprechend zu erhöhen, wodurch mehr Energie der Stoßwelle in höheren Frequenzbändern propagiert.

Dies kann z.B. durch Hochvoltkabel mit niedriger Induktivität erreicht werden, zum Beispiel durch ein kürzeres axiales Hochvoltkabel, insbesondere kürzer als 1,50m, vorzugsweise kürzer ca. 1,10m und noch vorzugsweise kürzer als 1,0m.

Grundsätzlich können piezoelektrische, elektromagnetische und elektrohydraulische Stoßwellengeneratoren eingesetzt werden. Bei schädelknochennahen Lokalisationen der Alzheimer Areale können auch ballistische Generatoren eingesetzt werden.

Jedoch ist eine nicht-repetitive, monotone Applizierung der Stoßwellen mit einer gewissen Variabilität von Stoßwelle zu Stoßwelle, d.h. alternierende Eigenschaften des Stoßwellenfeldes, zum Schutze der Zellen und Neuronen vorteilhaft. Günstig ist eine Variabilität der Amplituden der Druckverläufe von 5%, noch günstiger von 10%. Auch die Variabilität in der Frequenz der dominanten Frequenzbänder kann 2-5%, vorzugsweise 2-10% und noch vorzugsweiser betragen 2-15% betragen.

Die Aufgabe wird außerdem gelöst durch eine Vorrichtung für die extrakorporale Stoßwellentherapie, insbesondere für die Behandlung von Alzheimererkrankungen, insbesondere wie oben beschrieben, die mindestens einen, insbesondere elektrohydraulischen, Stoßwellengenerator und mindestens einen, insbesondere elektrohydraulischen, Stoßwellenapplikator umfasst. Der Stoßwellenapplikator umfasst mindestens den eigentlichen Therapiekopf, der an dem Patienten angelegt werden kann und insbesondere ein Verbindungskabel und einen Stecker.

Die Vorrichtung umfasst eine Impulssteuereinheit, die dazu ausgelegt ist, den elektrohydraulischen Stoßwellengenerator so einzustellen, dass die Druckverläufe der Stoßwelle idealerweise Anstiegszeiten von 6-50ns, bevorzugt 6-22ns, noch bevorzugter 9-12ns, aufweist. Solche kurzen Anstiegszeiten sind bevorzugt mit a priori Stoßwellen durch elektrohydraulische Stoßwellengeneratoren realisierbar.

Die Impulssteuereinheit kann dafür ausgelegt sein, die Anstiegszeit einzustellen. Eine kürzere bzw. längere Anstiegszeit lässt sich beispielsweise mit einer niedrigeren bzw. höheren Induktivität des Entladekreises erreichen.

Die Anstiegszeit hängt aufgrund des nichtlinearen Aufsteilungseffekt bei Stoß- bzw. Druckwellen auch von der Entladeleistung des Entladeschwingkreises bzw. der Amplitude der erzeugten Stoß-bzw. Druckwelle ab. Eine kürzere bzw. längere Anstiegszeit lässt sich mit einer höheren bzw. niedrigeren Entladeleistung des Entladekreises erreichen.

Es hat sich gezeigt, dass eine Fragmentation und eine Auslösung der Amyloid-Ablagerungen durch in kurzer Zeit aufgesteilte Stoßwellen erfolgt.

Ebenso können Tau-Fibrillen durch Aufbringen von Stoßwellen, die in Zeitspannen von 6 bis 16ns, vorzugsweise 8-10ns aufgesteilt sind, fragmentiert und ausgelöst werden.

Die Vorrichtung für die extrakorporale Stoßwellentherapie wie oben beschrieben, insbesondere eine elektrohydraulische Stoßwellenvorrichtung, eine Impulsfrequenzsteuereinheit umfassen, die dazu ausgelegt ist, den Stoßwellengenerator so einzustellen, dass Stoßwellen mit einer Impulsfrequenz (bzw. Impulsrate) größer 10 Hz, vorzugsweise größer 15 Hz abgegeben werden, vorzugweise mit einer Impulsfrequenz größer gleich 20 Hz, noch vorzugsweiser größer gleich 30 Hz , noch vorzugsweiser größer gleich 40 Hz, noch vorzugsweiser größer gleich 50 Hz und ganz besonders bevorzugt im Bereich von 50Hz bis 100 Hz .

Bevorzugt ist die Impulsfrequenzsteuereinheit dazu ausgelegt, die Entladungsrate eines elektrohydraulischen Stoßwellengenerators zu verändern. Es gibt technisch viele Möglichkeiten, die Impulsfrequenz zu verändern und es sei hier auf den breiten Stand der Technik in der Elektrotechnik verwiesen. Eine schnellere Aufladung des Kondensators zwischen zwei Pulsen kann beispielsweise durch ein größeres Ladegerät erfolgen.

Eine wichtige Rolle bei der Fragmentation und Auflösung von Amyloidablagerungen und von Tau-Fibrillen spielt die Dehnungsgeschwindigkeit gesunder Gehirnareale und der Proteinablagerungen. Die Dichte und die Steifigkeit der Proteinablagerungen sind signifikant höher als bei gesunden Gehirnarealen. Aus diesem Grund müssen im Frequenzspektrum der Stoßwellen, nach der Propagation durch die Schädeldecke relativ hochfrequente und pegelstarke Frequenzbänder im Bereich oberhalb 100kHz enthalten sein, vorzugsweise oberhalb 140kHz, noch vorzugsweiser oberhalb 180kHz, noch vorzugsweiser oberhalb 250 kHz. Denn die Rückdehnungsgeschwindigkeit der Amyloidablagerungen und der Tau-Fibrillen erfolgt schneller als bei gesunden Neuronen. Die zweite Stoßwelle sollte vor der vollständigen Rückdehnung der Amyloidablagerungen erfolgen. Die zweite und die darauf folgenden Stoßwellen bewirken weitere Dehnungen der Amyloidablagerungen bis zum Erreichen der letalen Dehnungen und der Fragmentation der schädlichen Proteinablagerungen.

Die Impulsfrequenzen bzw. Impulsraten der Stoßwellen werden rechnerisch bestimmt und in Versuchen mit Amyloidablagerungen validiert. Im Allgemeinen liegen die Frequenzen für die Alzheimer Behandlung zwischen 20 und 30 Hz. Bei einigen frühzeitig gebildeten Ablagerungen bei 40 Hz. Mit elektrohydraulischen Stoßwellengeneratoren sind hohe Impulsfrequenzen bis zu 100 Hz umsetzbar. Für die effektive Beseitigung der Amyloidstrukturen werden daher in der Therapie Stoßwellen-Impulsfrequenzen größer 15 Hz, vorzugweise 15 Hz bis 25 Hz, noch vorzugsweiser etwa 35 Hz bis 45 Hz und ebenfalls bevorzugt zwischen 45 Hz bis 55 Hz eingesetzt. Durch die hohe Impulsfrequenz kann die Leistung der einzelnen Stoßwelle abnehmen, was jedoch ebenfalls die Vermeidung von hohen Druckstößen und Gewebeschädigungen dient. Zusätzlich kann der zeitliche Abstand jeder einzelnen Stoßwelle zur Vorgänger-Stoßwelle und die maximale Amplitude jeder einzelnen Stoßwelle eingestellt werden. Auf diese Weise können beliebig lange Stoßwellen-Sequenzen realisiert werden. In den Sequenzen können beispielsweise die Amplituden der Stoß- bzw. Druckwellen ansteigen, die Impulsfrequenz kann steigen. Falls die Induktivitäten auch elektronisch veränderbar sind bzw. sich mit der Amplitudenhöhe oder Impulsfrequenz verändern, so können sich ggf. auch die Frequenzbänder ändern, in denen die Energie appliziert wird.

Mehrere gleiche oder verschiedene Stoßwellen-Sequenzen können dann hintereinander mit dem Applikator bzw. Therapiekopf appliziert werden.

Auch können bestimmte Muster von Stoßwellen, die sich aus einer KI-gestützten Analyse oder aus erfolgreichen klinischen Tests ergeben, verwendet werden. Auch können die Impulse mit zwei oder mehr Applikatoren bzw. Therapieköpfen gezielt aus verschiedenen Richtungen auf eine Gehirnregion gerichtet werden und damit die Dehnungen der Amyloidablagerungen erhöht, insbesondere auch die Scherdehnungen.

Amyloidablagerungen werden nach 4-5 Stoßwellenbehandlungen vollständig fragmentiert und aufgelöst. Die Behandlungen sollten täglich, jedoch nicht seltener als ein bis zweimal wöchentlich erfolgen. Die Stoßwellenbehandlungen können alle 4-5 Tage wiederholt werden.

Pro Behandlung können 1000 bis 4000 Stoßwellen, vorzugsweise 2000-3000 Stoßwellen, aufgebracht werden.

Die Vorrichtung für die extrakorporale Stoßwellentherapie kann einen Therapiekopf aufweisen, der so ausgelegt ist, dass die Vorrichtung schwach fokussierte oder unfokussierte oder divergierende Stoß- oder Druckwellen emittieren kann.

Mit gering fokussierten, unfokussierten oder divergierenden Stoßwellen können lokale Schädigungen des Gehirns vermieden werden.

Die Vorrichtung, insbesondere eine elektrohydraulische Stoßwellenvorrichtung, kann eine Fokussteuereinheit und mindestens einen Applikator, bevorzugt mit einem Reflektor und einem Elektrodenpaar, umfassen. Die Fokussteuereinheit kann dazu ausgelegt sein, die abgegebenen Stoßwellen zu fokussieren und zu defokussieren. Insbesondere kann die Fokussteuereinheit dazu ausgelegt sein, die Position mindestens einer Elektrode im Reflektor zu verschieben oder eine Elektrode gegen eine Elektrode mit abweichender Geometrie (andere Position des Elektrodenspalts) auszutauschen.

Speziell bei den elektrohydraulischen Stoßwellengeneratoren kann auch durch die Art des Reflektors, insbesondere die Form, ein sehr großer Einfluss auf das induzierte Stoßwellenfeld genommen werden. Bekanntlich werden bei ellipsoiden Reflektoren die Stoßwellen stark fokussiert, wenn die Entladung im Fokuspunkt F1 stattfindet. Bei Paraboloid-Reflektoren ist die Fokussierung in der Regel viel schwächer bzw. der Fokus F2 im Unendlichen, falls die Entladung im Paraboloid-Fokus erfolgt. Die axiale Länge der Reflektoren im Vergleich zum größten Durchmesser ist ebenfalls ein Einflussfaktor für die Fokussierung wie auch die Position der beiden Elektroden bzw. der Elektrodenspalt. Durch die axiale Verlagerung der Position des Elektrodenspalts z.B. in Richtung auf den Reflektoraustritt kann eine gering fokussierte oder unfokussierte Stoßwellenpropagation erfolgen. Im Extremfall ist auch eine divergierende Stoßwellenpropagation möglich. Hierfür sind u.a. paraboloide und kegelförmige Reflektoren verwendbar. Auch sogenannte Freiform-Reflektoren können eingesetzt werden. Die physikalische wirksame axiale Länge des Reflektors (Innenseite) beträgt vom Reflektorrand vorteilhafterweise 40mm, noch vorteilhafterweise kleiner gleich 35mm bzw. noch vorteilhafterweise kleiner 30mm.

Es zeigt sich durch Simulationen, dass bei einem Verhältnis von axialer Reflektor-Länge zu größtem Reflektor-Innendurchmesser (es geht hier nur um den akustisch relevanten innenliegenden Teil des Reflektors) von ungefähr 1.1 bis 0.9 eine deutliche Aufweitung stattfindet. Bevorzugt ist ein Verhältnis von 0.9 bis 0.8, noch weiter bevorzugt ein Verhältnis von 0.8 bis 0.7, noch weiter bevorzugt ein Verhältnis von 0.7 bis 0.5.

Zusätzlich kann der Rand der Reflektoren abgerundet werden, vorzugsweise mit einem Radius von 3mm bis 5mm, noch vorzugsweiser mit einem Radius von 6mm bis 10mm. Die Abrundung bewirkt eine geringere Streuung.

Durch einen Versatz der Elektroden und somit der Position der Entladung zu dem geometrischen Fokuspunkt des Ellipsoids bzw. dem Fokuspunkt des Paraboloids lässt sich ebenfalls die Fokussierung reduzieren. Hierbei kann ein Abstand von 1mm bis 10mm vom Fokus zielführend sein, ein Abstand von 2mm bis 8mm bevorzugt, bzw. ein Abstand von 3-7mm weiter bevorzugt.

Grundsätzlich vorteilhaft ist, wenn der Abstand zum geometrischen Fokus in Richtung der zu applizierenden Stoßwellen einstellbar ist. Auch durch eine gezielte asymmetrische Position der Elektroden oder eine Asymmetrie des Reflektors kann die Fokussierung ebenfalls reduziert werden. Bekanntlich führen konusförmige Wellenleiter zu einer sphärischen Wellenausbreitung. Auch kegelförmige oder kegelstumpfförmige Reflektoren sind daher zielführend für eine schwache Fokussierung. Wenn die Entladung möglichst nahe am Schnittpunkt der axialen Kegelmantellinien stattfindet, kann man zudem Stoßwellen erzeugen, die im Wesentlichen nur eine sphärische Primär-Druckwelle besitzen.

Eine sphärische Stoßwelle kann günstig sein, da die Stoßwellen zusätzlich durch die rundliche Form des Schädelknochens und die höhere Schallgeschwindigkeit im Schädelknochen fokussiert werden. Somit kann nur bei einer vom Applikator bzw. Therapiekopf ausgesandten, leicht divergierenden Stoß- oder Druckwelle beim Auftreffen auf den gebogenen Schädel im Gehirn eine praktisch planare und gleichmäßige und breite Wellenfront (ungefähr in der Breite des halben bis ganzen Reflektordurchmessers) innerhalb des Schädels erreicht werden und weite Bereiche im Gehirn schonend behandelt werden.

Die Elektrodenposition des Innen- und/oder Außenleiters kann derart optimiert werden, dass unter Berücksichtigung der Schädelkrümmung erreicht wird, dass planare Wellen im Behandlungsbereich propagieren (dies wird z.B. bei ellipsoiden Reflektoren erreicht, wenn der Elektrodenspalt außerhalb des Fokus F1 liegt). Die Elektrodenposition des Innen- und/oder Außenleiters kann manuell erfolgen bzw. durch Auswechseln der Elektrode oder auch durch einen Verstellmechanismus, bei dem mindestens eine Elektrodenposition verstellt wird.

Unter Berücksichtigung der Schädelkrümmung können auch mit einer vom Applikator bzw. Therapiekopf radial abgestrahlten divergenten Stoß- oder Druckwelle planare, nicht fokussierte Wellen im Behandlungsbereich erreicht werden.

Falls erforderlich können mit stark divergierenden Stoß- oder Druckwellen, die auf den Schädel treffen, im Schädel auch divergierende Wellen erreicht werden, die gegenüber den planaren Stoß- oder Druckwellen noch niedrigere Energiekonzentrationen aufweisen.

Umgekehrt ist der Fokussierungseffekt durch die runde Form und höhere Dichte bzw. Festigkeit des Schädels u.U. ein weiteres (bislang möglicherweise stark unterschätztes) Risiko bei der Applizierung fokussierter Stoß- bzw. Druckwellen.

Die Fokussteuereinheit kann dazu ausgelegt sein, Daten über den Schädel, insbesondere zur Dichte und/oder Geometrie, zu empfangen und bei der Fokussierung bzw. der Einstellung der Fokussierung der Stoßwellen zu berücksichtigen. Die lokale Schädelstruktur kann zu einer stärken Fokussierung oder zu einer Defokussierung der emittierten Stoßwelle führen.

Die Fokussteuereinheit kann dazu ausgelegt sein, die Amplitude zu variieren, so dass gering fokussierte oder nicht-fokussierte Stoßwellen emittiert werden, die eine Variabilitität bzgl. Amplitude der Stoß- bzw. Druckwellen aufweisen.

Das Verhältnis des Absolutbetrages der positiven zum Absolutbetrag der negativen Druckamplitude, abs(p+)/abs(p-), ist grösser als 1, vorzugsweise größer 2, noch vorzugsweiser größer 3.

Die Amplituden-Variabilität wird als Abweichung von Mittelwert der maximalen Stoß- oder Druckwellendruckamplitude charakterisiert. Eine Amplituden-Abweichung von bis zu 5%, vorzugsweise bis zu 10% und noch vorteilhafter vorzugsweiser von 11-25% ist vorteilhaft.

Analog lässt sich auch eine Anstiegszeit-Variabilität charakterisieren. Die Anstiegszeit, die bei der Stoß- oder Druckwelle als Zeitraum definiert ist, in dem die Amplitude von 10% des Maximalwertes auf 90% des Maximalwertes (p+) ansteigt, kann von einem mittleren Wert der Anstiegszeit abweichen. Eine Anstiegszeit-Abweichung von bis zu 10%, vorzugsweise bis zu 20% und noch vorteilhafter 20-50% ist vorteilhaft.

Hier bieten gerade die elektrohydraulischen Stoßwellengeneratoren einen weiteren Vorteil.

Eine inhärente Variabilität der alternierenden Amplitude der Stoßwellen ergibt sich durch die schussweise variierende Position des Plasma-Kanals bzw. der Gasblase zwischen den Elektroden. Dadurch kommt es bei wiederholt applizierten, alternierenden Stoßwellen zu einer weiteren lokalen Abmilderung bzw. zeitlichen Defokussierung der der Stoßwellen im Schädel über die Zeitdauer der Behandlung an einer Stelle. Es wird dadurch vermieden, dass an gleicher Stelle durch monotone Wiederholung sehr ähnlicher bzw. (nahezu) identischer Druckpulse mit geringer Streuung (kleine Variabilität bzw. wenig alternierende Eigenschaften des Stoß- bzw. Druckwellenfeldes) lokal Gewebe- bzw. Zellstrukturen u.U. sehr viel schneller geschädigt werden können. Somit ist die Variabilität der alternierenden Druckverläufe der elektrohydraulischen Stoßwellengeräte ein positives Merkmal im Hinblick auf eine möglichst schonende Behandlung von Gehirngewebe.

Die Erfindung ermöglicht somit gleichmäßig verteilte Druckfelder im gesamten Gehirnareal und vermeidet Druckspitzen, insbesondere monoton wiederholte lokale Druckmaxima, im Fokus-Areal. Diese Vorteile gelten insbesondere für elektrohydraulische Stoßwellengeneratoren.

Eine gleichmäßige Verteilung der Stoßwellenbeaufschlagung kann auch erreicht werden, wenn die Vorrichtung zwei oder mehr Stoßwellenapplikatoren bzw. Therapieköpfe, die gleichzeitig Stoßwellen emittieren können, umfasst. Insbesondere kann die Vorrichtung zwei oder mehr Entladekondensatoren und zwei oder mehr Stoßwellenapplikatoren bzw. Therapieköpfe mit jeweils einem Elektrodenpaar umfassen, wobei insbesondere alle Elektrodenpaare gleichzeitig oder nahezu gleichzeitig durch die Entladekondensatoren zündbar sind.

Gegebenenfalls kann auch nur ein Entladekondensator verwendet werden (insbesondere bei eine Entladeschaltung des Kondensators).

Insbesondere können die einzelnen Elektrodenpaare zeitlich versetzt gezündet werden und jeweils unterschiedliche Sequenzen von Stoß- oder Druckwellen erzeugt werden.

Die Stoßwellen der Stoßwellenapplikatoren bzw. Therapieköpfe können sukzessive gezündet werden, so dass beispielsweise in schneller Abfolge nacheinander Stoß- oder Druckwellen in dem Behandlungsgebiet aus verschiedenen Richtungen ankommen.

Die Vorrichtung für die extrakorporale Stoßwellentherapie kann eine Steueranordnung umfassen, wobei die Steueranordnung dazu ausgelegt ist, einen Betriebsmodus zur Behandlung von Alzheimererkrankungen einzunehmen und in diesem vorbestimmte Einstellparameter für die Frequenzsteuereinheit, die Impulssteuereinheit und/oder der Impulsfrequenzsteuereinheit festlegt.

Die Vorrichtung kann bei Bedarf auch für andere Gewebe und Indikationen eingesetzt werden, kann aber auch wieder für die Behandlung von Alzheimererkrankungen eingerichtet werden.

Die Aufgabe wird außerdem gelöst durch ein System zur Behandlung von Alzheimererkrankungen umfassend eine Vorrichtung für die extrakorporale Stoßwellentherapie wie oben beschrieben. Das System umfasst außerdem eine Prozessoreinheit, die dazu ausgelegt ist, Diagnosedaten von einem Datenspeicher oder einer Diagnosevorrichtung, zu empfangen, Einstellparameter für die Vorrichtung für die extrakorporale Stoßwellentherapie in Abhängigkeit der Diagnosedaten zu bestimmen, die Einstellparameter an die Vorrichtung für die extrakorporale Stoßwellentherapie abzugeben.

Bei der Diagnosevorrichtung kann es sich um eine Bildgebungsvorrichtung (mit entsprechender Prozessoreinheit) oder eine Vorrichtung zur Analyse von Blutwerten im Hinblick auf Amyloid handeln.

Das System kann eine Diagnosevorrichtung und/oder einen Datenspeicher umfassen.

Ein sogenannter Precivity AD Bluttest kann Amyloid detektieren und für die Beurteilung des Behandlungserfolgs herangezogen werden.

Die Stoßwellenbehandlungen kann gesteuert werden auf Basis von Messungen der Blutwerte im Hinblick auf Amyloid.

Wenn mittels Bluttest kein Amyloid mehr festgestellt werden, kann die Behandlung beendet werden. Wenn weniger oder mehr Amyloid als bei einer früheren Diagnose festgestellt wird, können die Einstellparameter bei der Stoßwellenbehandlung entsprechend angepasst werden.

Die Diagnosedaten können Daten zur Dichte und/oder Geometrie des Schädels umfassen. Weiterhin können auch andere ermittelte oder berechnete lokale physikalische Parameter des Gehirngewebes wie z.B. Ausrichtung bzw. Anisotropien, Struktur, Festigkeit, Schwingungs- und Dämpfungsverhalten, Zellart, Zellformen verwendet werden, um erkrankte Zellbereiche des Gehirns zu detektieren bzw. auch den Behandlungserfolg zu kontrollieren.

Die lokal veränderliche Schädelkrümmung, -dicke, -dichte und - festigkeit kann für die Planung der gewünschten Stoßwellen-Charakteristik mit einbezogen werden.

Im Falle der Behandlung anderer Körperteile können die Diagnosedaten entsprechend Daten zur lokalen Dichte, Festigkeit, Struktur, Schwingungs- und Dämpfungsverhalten und/oder Geometrie von Gewebe, Organen und Knochen umfassen.

Unter Berücksichtigung der lokalen Schädelkrümmung können beispielweise mittels einer vom Stoßwellenapplikator abgegebenen divergenten oder radial abgestrahlten Stoßwelle planare, nicht fokussierten Druckwellen im Behandlungsbereich erzeugt werden. Im Extremfall können auch divergente Stoßwellen im Schädel erzeugt werden.

Beispielweise kann die Steuervorrichtung dazu ausgelegt sein, die Elektrodenpositionen einer elektrohydraulischen Stoßwellenvorrichtung derart einzustellen, dass unter Berücksichtigung der Schädelkrümmung planare Wellen im Behandlungsbereich propagieren.

Die Einstellparameter sind insbesondere ausgewählt aus der Gruppe
- Anzahl, Position und/oder Ausrichtung der Stoßwellenapplikatoren bzw. Therapieköpfe,
- Entladefrequenz des Entladeschwingkreises,
- Anstiegszeit der Stoßwelle,
- Leistungsstufe bzw. Ladespannung des Kondensators,
- Fokussierungsgrad,
- Impulsfrequenz (bzw. Impulsrate),
- Anzahl der zu applizierenden Stoßwellen,
- Sequenzen der einzelnen Stoßwellen,
- Wiederholrate der Sequenzen.

Für die Ermittlung von Einstellparametern können bevorzugt hochaufgelöste Patienten-Daten (CRT, MRT etc.) eingelesen werden (z.B. in DICOM).

Hochaufgelöst kann in diesem Zusammenhang bedeuten, dass eine Auflösung bis auf Zellebene stattfindet.

Die Patienten-Daten können für eine FEM-Berechnung aufbereitet werden (z.B. mit MATLAB) und mind. eine FEM-Berechnung (z.B. mit ANSYS) kann zur Simulation des Einflusses der Stoßwellen und/oder Stoßwellensequenzen auf Zellebene durchgeführt werden.

Mit dieser Vorgehensweise können Einstellparameter für eine effektive und gewebeschonende Behandlung abgleitet werden.

Das System kann einen Datenspeicher für die Speicherung von Patientendaten, Berechnungsdaten und Behandlungsparametern umfassen.

Die Steueranordnung kann dazu ausgelegt sein, ermittelten Behandlungsparameter abzuspeichern und/oder bei einer Behandlung die für einen Patienten optimalen lokalen Behandlungsparameter auszulesen.

Um patientenindividuelle Stoßwellen in dem AD-Areal aufzubringen, können beispielsweise CT/MRT Patientendaten (z.B. mit CRT oder MRT-Geräten gewonnen) in ein DICOM/MATLAB/FEM-Programmsystem eingelesen werden, über MATLAB in ein FEM-Berechnungsmodell (ANSYS, PZFLEX) konvertiert werden und die Stoßwellenpropagation vom Applikator (ggf. auch bei unterschiedlichen Leistungsstufen, Richtungen, spektraler Energieverteilung) durch den Schädel ins Gehirn berechnet bzw. numerisch gelöst werden.

Neben der Dichte, Wellengeschwindigkeiten und Absorptionskoeffzienten von Schädel und Gehirn werden auch lokale Struktureigenschaften im Gehirn bei der Simulation detailliert berücksichtigt. Die Ergebnisse der Simulation der Stoßwellenpropagation bestimmen die Behandlungsparameter, wie z.B. Richtung bzw. den Bereich der Behandlung, die Höhe der einzustellenden Leistung bzw. die daraus resultierenden Druckamplituden, die Frequenz und zeitliche Abfolge bzw. Sequenzen der Einzelimpulse sowie die tieffrequenten Frequenzbänder, über die bevorzugt die Energie übertragen wird.

Dabei kann auch das lokale Schwingungsverhalten des Schädelknochens berücksichtigt werden, welches sich aus der Dicke des Schädelknochens, dessen Formgebung, seiner lokalen Dichte- und Steifigkeitswerte ergibt.

Zudem können auch noch weitere patientenspezifische Parameter oder Diagnosedaten wie z.B. Geschlecht, Alter, geometrischer Werte (Gehirngröße, Volumen, Dichte, Schädeldicke sowie Schädeldichte und Schädelfestigkeit), hochaufgelöste Scans von auffälligen Gehirnarealen, sowie die lokal im Gehirn hervorgerufenen Wirkung bei der Berechnung der Wellenpropagation bei der Ableitung von Behandlungsparameter berücksichtigt werden.

Durch den Einsatz künstlicher Intelligenz bzw. Deep Learning können z.B. anhand von patientenindividuellen Daten (CT, MRT etc.) vor und nach der einer oder mehrerer durchgeführten Behandlung, anhand von Simulationsrechnungen, ggf. anhand von Messungen (z.B. Blutmessungen), sowie anhand festgestellter Behandlungsverläufe und -erfolge, die bestgeeigneten Parameter für die Stoßwellen-Behandlungen bei AD berechnet werden.

Bei der Auswertung der von Simulationsergebnissen sowie CT/MRT Patientendaten vor und nach einer oder mehreren Behandlungen von einer Vielzahl von Patienten kann künstliche Intelligenz verwendet werden.

Bei Auswahl bzw. Zuordnung von Behandlungsparametern zu patientenindividuellen Daten kann künstliche Intelligenz eingesetzt werden. Die ermittelten Behandlungsparameter sollten dabei jedoch vor einer Behandlung stets von einem Arzt geprüft werden.

Die Diagnosevorrichtung kann eine Bildgebungsvorrichtung für 3d-Darstellungen des Kopfes sein und die Prozessoreinheit dazu ausgelegt sein, Propagationen von Stoßwellen in einem Kopf aufgrund der Bilddaten, z.B. auf Basis von CT/MRT Patientendaten, zu simulieren und in Abhängigkeit der Simulationsergebnisse optimierte Einstellparameter für die Stoßwellenbehandlung zu bestimmen.

Im Rahmen dieser Anmeldung sind CT und MRT stellvertretend für alle anwendbaren 2D und/oder 3D-Bildgebungs-Verfahren angeführt.

DICOM ist ein aktueller internationaler Standard, der zur Speicherung medizinischer Bilder wie CT- und MRI-Scans verwendet wird und steht hier ebenfalls stellvertretend für alle anderen vergleichbaren Standards in diesem Bereich der Medizin.

MATLAB ist eine Software zur Lösung mathematischer Probleme und zur grafischen Ergebnisdarstellung und steht stellvertretend für alle weiteren Berechnungs-Softwareanwendungen.

FEM steht für Finite-Element-Analyse und ist eine Simulationsmethode, bei der die Bereiche eines Berechnungsgebietes (hier im Kontext der Schädel mit dem Gehirn) in kleine Elemente (die finiten Elemente) unterteilt wird, um das physikalische Verhalten in den relevanten (Teil-)Bereichen, hier im Kontext bei der Applizierung von Stoß- oder Druckwellen, vorherzusagen. Auch die FEM steht stellvertretend für alle weiteren numerischen oder auch analytischen Berechnungsmethoden, die in diesem Zusammenhang relevant sind oder sein werden. Alternativ zur eigentlichen FEM-Software können zum Beispiel auch Finite-Differenz-Berechnungsverfahren verwendet werden. ANSYS, PZFLEX etc. sind beispielhafte Programme für FEM-Berechnungen.

Für die mathematische Beschreibung der komplexen Vorgänge der Stoßwellenpropagation sind FEM Simulationsmodelle besonders geeignet. Auf der Basis der Ergebnisse ist eine "Vorhersage" der Ausbreitung der Druckwelle im Gewebe und der Wirkung auf Amyloidstrukturen möglich. Dieses patientenindividuelle Verfahren basiert auf der Berücksichtigung der einzelnen anatomischen Strukturen, Mikrostrukturen wie Filamente und Mikrotubuli und den physikalisch-akustischen Impedanzgesetzen. Durch den Einsatz der FEM-Simulationen wird die größtmögliche Sicherheit aus Sicht des Patienten erreicht, insbesondere Zell- und Gewebeschäden werden durch die schwach fokussierten bzw. unfokussierten Stoßwellen wirksam vermieden.

Im Rahmen dieser Anmeldung wird von DICOM/MATLAB/FEM-Programmsystem gesprochen und damit werden alle Programmsysteme gemeint, die Patientendaten einlesen, in ein Berechnungsmodell überführen und numerisch mit einer Software auswerten.

Mit DICOM/MATLAB/FEM-Simulationsmodell wird das entsprechende Simulationsmodell eines DICOM/MATLAB/FEM-Programmsystem bezeichnet, welches auch stellvertretend für allgemeine Simulationsmodelle vergleichbarer Programmsysteme steht.

Das System kann über ein Ortungssystem verfügen, mit dem der mindestens eine Stoßwellenapplikator bzw. dessen Therapiekopf auf die Amyloid-Areale ausgerichtet wird. Die Ausrichtung kann manuell erfolgen oder auch durch eine automatische Positionierungseinrichtung.

Das Ortungssystem kann einen Patienten Rigid Body, eine Steuerung der optischen Ortung, einen Applikator Rigid Body und eine DICOM-MATLAB-FEM Schnittstelle der Stoßwellenpropagation umfassen. Mit Ridid Body ist eine Starrkörper-Repräsentation des Patienten (oder Teile dessen) bzw. des Applikators etc. gemeint. Hierzu werden am Patientenkopf und am Therapiekopf entsprechende Marker appliziert, durch die die genaue Position und Ausrichtung des Patientenkopfs und des Therapiekopfs ermittelt werden kann.

Die meisten Aspekte der Erfindung beziehen sich nicht nur auf die Behandlung von Gehirngewebe. Auch andere Körperbereiche können prinzipiell auf analoge Art behandelt werden. Z.B. sind Darmzellen in einigen Aspekten ähnlich zu Gehirnzellen. Eine schonende Applizierung ist auch in diesem und anderen Körperbereichen sinnvoll. Dank der schwach bzw. unfokussierten Stoßwellen können auch große Körperbereiche gleichmäßig behandelt werden. Ebenfalls sind tierische Gehirne oder Körperbereiche behandelbar.

Das System kann eine Ultraschallvorrichtung und eine Kombinations-Steueranordnung umfassen, die dazu ausgelegt ist, Ultraschallwellen und Stoßwellen zeitnah in denselben Behandlungsbereich zu applizieren und insbesondere die Einstellparameter des Ultraschalls und der Stoßwellen aufeinander abzustimmen.

Das amyloid-destruktive Potential der Stoßwellenvorrichtung kann durch die stoßwelleninduzierte Aktivierung von Ultraschallsensibilatoren, nämlich Sonosensibilatoren die durch Ultraschall aktiviert werden (siehe auch: Lin et al., Ultrasound Activated Sensitizers and Applications, 2019, Angew. Chem. Int. Ed. 10.1002/anie. 201906823), verbessert werden. Dabei werden insbesondere Kavitation und thermische Energie zur Auslösung verwendet. Insbesondere können stoßwellenspezifische Ultraschallpulse (bzw. Stoßwellen, die Ultraschallanteile besitzen) realisiert werden. Grundsätzlich können für die Auslösung der Ultraschallsensibilatoren Stoßwellen und Ultraschallwellen auch gleichzeitig verwendet werden.

Das System kann eine Medikamentenabgabevorrichtung umfassen. Die Prozessoreinheit kann dazu ausgelegt sein, Abgabeparameter für die Medikamentenabgabevorrichtung in Abhängigkeit der Diagnosedaten zu bestimmen und die Abgabeparameter an die Medikamentenabgabevorrichtung abzugeben.

Alternativ kann die Medikamentenabgabevorrichtung auch nur dazu ausgelegt sein, die optimale Medikamentenabgabe in Form der Abgabeparameter anzuzeigen, um dann die Medikamente manuell zu verabreichen.

Die Alzheimer Behandlung per Stoßwelle kann als Kombinationstherapie mit monoklonalen Antikörpern wie dem neuen Wirkstoff Aducanumab bzw. Aduhelm (USA, zugelassen seit 2021) eingeplant werden.

Das System kann eine Temperiervorrichtung, insbesondere einen Kühlvorrichtung, für den Kopf des Patienten umfassen und die Prozessoreinheit kann dazu ausgelegt sein, Temperierparameter für die Temperiervorrichtung in Abhängigkeit der Diagnosedaten zu bestimmen und die Temperierparameter an die Temperiervorrichtung abzugeben.

Bei der Behandlung möglicherweise entstehende Wärme durch die Absorption von akustischen Wellen kann über die Kühlvorrichtung abtransportiert werden. Auf diese Weise kann die Therapie schonend erfolgend, und insbesondere ohne ungewollte Erwärmung der zu behandelnden als auch der nicht zu behandelnden oder benachbarten Areale.

Die Aufgabe wird ausserdem gelöst durch ein Verfahren zum Bereitstellen von Einstellparametern für eine Behandlung von Alzheimererkrankungen mit einem System wie oben beschrieben. Das Verfahren umfasst die Schritte Bereitstellen von Diagnosedaten von einem Datenspeicher und/oder einer Diagnosevorrichtung, sowie Bestimmen von Einstellparameter für die Vorrichtung für die extrakorporale Stoßwellentherapie in Abhängigkeit der Diagnosedaten.

Die Diagnosevorrichtung ist insbesondere eine Bildgebungsvorrichtung oder einer Vorrichtung zur Analyse von Blutwerten hinsichtlich Amyloid.

Das Verfahren umfasst außerdem insbesondere den Schritt Bestimmen von Einstellparameter für eine Ultraschallvorrichtung (insbesondere Stoßwellengenerator und Applikator mit Therapiekopf), Bestimmen von Abgabeparametern für eine Medikamentenabgabevorrichtung und/oder Bestimmen von Kühlparametern für eine Kühlvorrichtung.

Die Aufgabe wird außerdem gelöst durch ein Computerprogramm mit Programmcode zur Durchführung der Schritte des oben genannten Verfahrens, wenn das Programm auf einer Prozessoreinheit eines Systems wie oben beschrieben ausgeführt wird.

Die Aufgabe wird außerdem gelöst durch ein Computerprogrammprodukt, das direkt in einen internen Speicher eines Digitalcomputers geladen werden kann und das Softwarecodeabschnitte umfasst, die die Verfahrensschritte des ob genannten Verfahrens ausführen, wenn das Programm auf dem Digitalcomputer eines Systems ausgeführt wird, wie es oben beschrieben ist.

Patientenindividuelle Simulationsanalysen auf Basis von CRT, MRT etc. ermöglichen gleichmäßig verteilte Druckfelder im AD Areal. Eine zusätzliche Ortung ist nicht unbedingt erforderlich. Dennoch ist die Steuerung der Vorrichtung so ausgelegt, dass die in der Halterung flexibel integrierten Applikatoren mit Hilfe eines optischen Ortungssystems auf das AD-Areal ausgerichtet werden können und die Stoßwellen mit dem Applikator bzw. Therapiekopf gezielt mit berechneten Parametern appliziert werden.

Die Stoßwellenbehandlungen ist bei richtiger Anwendung gewebeschonend und können wiederholt werden, bis alle Proteinablagerungen aufgelöst sind und anti-inflammatorische und regenerative Prozesse eingeleitet werden.

Die erforderliche aufgebrachte Gesamtenergie und alle anderen Behandlungsparameter wie Energieflußdichte, Frequenz, Anzahl und Reihenfolge der Stoßwellen können rechnerisch festgelegt und experimentell validiert werden.

Die Zellmechanik kann sowohl von der zytoskelettalen Architektur der Zelle als auch von der Mechanik der größten Organelle in der Zelle, dem Kern, bestimmt werden.

Die Angaben in der Fachliteratur zu der Steifigkeit von Proteinablagerungen gehen weit auseinander. Aus diesem Grund sollten individuelle AFM (Atomic Force Microscopy) Messungen der Proteinablagerungen und elastographische Messungen eingesetzt werden. Sie ermöglichen die Bestimmung von patientenindividuellen Behandlungsparametern und dienen der Patientensicherheit. Es können jedoch bei genügend genauer Datenlage auch typische mechanische Parameter der Proteinablagerungen ermittelt und gespeichert werden und dann Bereichen im Patientengehirn anhand der vorhandenen CRT/MRT Daten zugeordnet werden. Dies beschleunigt die Bestimmung der Behandlungsparameter.

Bei der AD-Behandlung kann zum einen der Fokus auf der Zerstörung der Amyloid-Strukturen gelegt werden. Ein anderer Ansatz ist es, mit relativ geringen Druckamplituden bzw. Intensitäten bzw. Energieflussdichten (EFD) und einem unfokussierten oder schwach fokussierten Applikator vorhandene Entzündungen im Gehirn wirksam zu behandeln und damit die Entzündungen zu vermindern bzw. sogar zu eliminieren.

Bei der Behandlung mit schwach fokussierten oder unfokussierten Applikatoren ist aufgrund der geringeren Intensitäten im Behandlungsbereich keine aufwändige Ortung notwendig. Die Ortung wird noch überflüssiger, wenn zusätzlich auch noch die Leistungsstufe reduziert wird, d.h. die Kondensatorspannung reduziert wird oder eine hohe Impulsfrequenz (bzw. Impulsrate), z.B. im Bereich von 20-100Hz verwendet wird.

Weitere Informationen sowie mehrere vorteilhafte Ausgestaltungen der Erfindung werden nachfolgend anhand von Abbildungen/Figuren verdeutlicht. Funktionell gleiche Elemente sind in der Regel mit den gleichen Bezugszeichen versehen.

Es zeigen
- Figur 1: eine schematische Darstellung eines ersten Beispiels für eine erfindungsgemäße Vorrichtung;
- Figur 2: eine schematische Darstellung eines zweiten Beispiels für eine erfindungsgemäße Vorrichtung;
- Figur 3: eine schematische Darstellung des Therapiekopf eines Applikators einer elektrohydraulischen Stoßwellenvorrichtung;
- Figur 4: Darstellungen eines elektromagnetischen Therapiekopfes eines Applikators;
- Figur 5: eine Darstellung eines weiteren Therapiekopfes eines Therapiekopfes eines Applikators und Druckverläufe;
- Figur 6: ein erstes Beispiel für ein Frequenzspektrum eines Druckverlaufs;
- Figur 7: ein zweites Beispiel für ein Frequenzspektrum eines Druckverlaufs;
- Figur 8: eine schematische Darstellung eines dritten Beispiels für eine erfindungsgemäße Vorrichtung in Form einer ringförmigen Struktur mit montierten Therapieköpfen mehrerer Applikatoren;
- Figur 9: eine schematische Darstellung eines vierten Beispiels für eine erfindungsgemäße Vorrichtung in Form einer ringförmigen Struktur mit montierten Therapieköpfen mehrerer Applikatoren;
- Figuren 10a, 10b: zelluläre Strukturen vor und nach der Applizierung einer Stoßwelle;
- Figur 11: eine Darstellung des Ergebnisses einer FEM-Simulation der Applizierung einer Stoßwelle auf zelluläre Strukturen;
- Figur 12: eine Darstellung zelluläre Strukturen krankhafter Proteinablagerungen in AD-Arealen;
- Figur 13: eine Darstellung von Dehnungsfeldern in zellulären AD-Strukturen;
- Figuren 14a, 14b: Darstellungen von FEM-Simulationsanalysen der Applizierung von Stoßwellen auf AD-Strukturen;
- Figur 15: ein fünftes Beispiel für ein erfindungsgemäßes System;
- Figur 16: ein erstes Beispiel für die Applizierung einer Stoßwelle auf ein Kopfareal;
- Figuren 17a, 17b: weitere Beispiele die Applizierung von Stoßwellen auf ein Kopfareale;
- Figuren 18a, 18b: weitere Beispiele für die Applizierung von Stoßwellen auf einen Kopf;
- Figuren 19a, 19b: weitere Beispiele für die Applizierung von Stoßwellen auf einen Kopf;
- Figuren 20a, 20b: einen Druckverlauf einer Stoßwelle und das zugehörige Frequenzspektrum;
- Figur 21: ein beispielhaftes Ersatzschaltbild des Entladeschwingkreises eines Stoßwellengenerators
- Figur 22: eine beispielhafte Darstellung des Zusammenhangs zwischen Kondensator-Entladefrequenzen und der Länge des koaxialen Hochspannungskabels;
- Figuren 23a-23g: Beispiele für Stoßwellengeräte mit Maßnahmen zur Änderung der Induktivitäten des Entladekreises;
- Figuren 24a, 24b: Reflektoren mit Elektroden in zwei verschiedenen Positionen;
- Figuren 25a, 25b: schematische Fokussierungseffekte bei der Applikation von Stoßwellen auf einen Kopf;
- Figur 26: schematisch Schritte eines erfindungsgemässen Verfahrens.

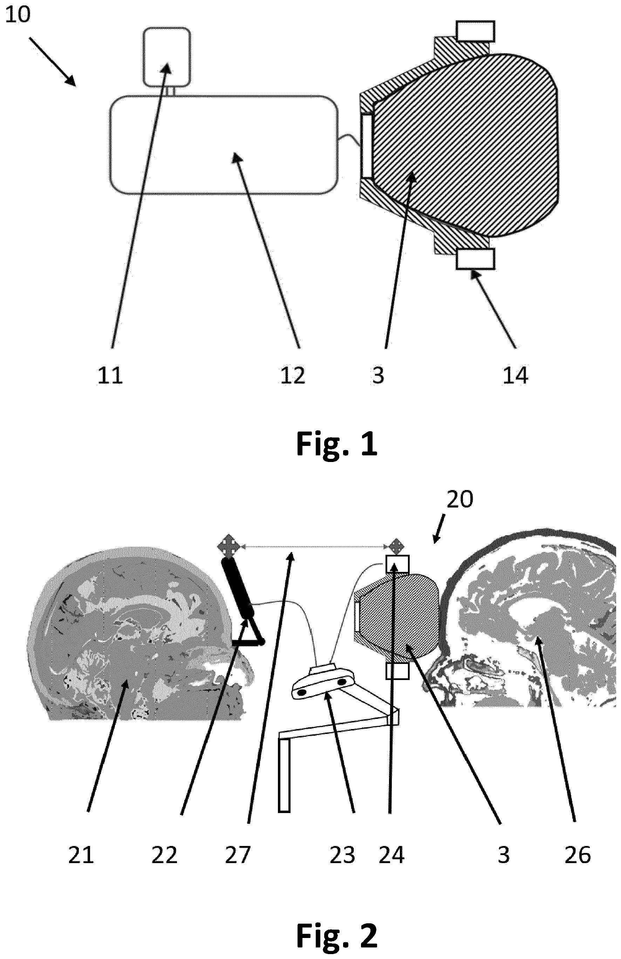

Fig. 1 zeigt eine schematische Darstellung eines ersten Beispiels für eine erfindungsgemäße Vorrichtung 10. Es sind die typischen Komponenten der Vorrichtung für die extrakorporale, gewebeschonende Behandlung von Alzheimer Erkrankungen mit Hilfe alternierender mechanischer Felder dargestellt. Zu sehen sind die Steuervorrichtung 11 der kapazitiven Entladung, der Stoßwellengenerator 12, Stoßwellen-Applikatoren 3 mit Übertragungsmedium, sowie ein Positionierungsmechanismus 14. Der Positionierungsmechanismus 14 stellt sicher, dass die flächige Applizierung der fokussierten, niedrigfokussierten, planaren, sphärischen bzw. divergierenden Stoßwellen an den relevanten Stellen im Gehirn erfolgt. Hierzu wird auf das DICOM-MATLAB-FEM-Programmsystem zurückgegriffen, welches durch detaillierte Analyse, die die lokale Zelleigenschaften und -verhalten berücksichtigt, die exakten Behandlungsparameter ermittelt, wie z.B. die Höhe der positiven und negativen Stoßwellenamplitude sowie die Wiederholfrequenz und ggf. auch die Anzahl der Stoßwellen.

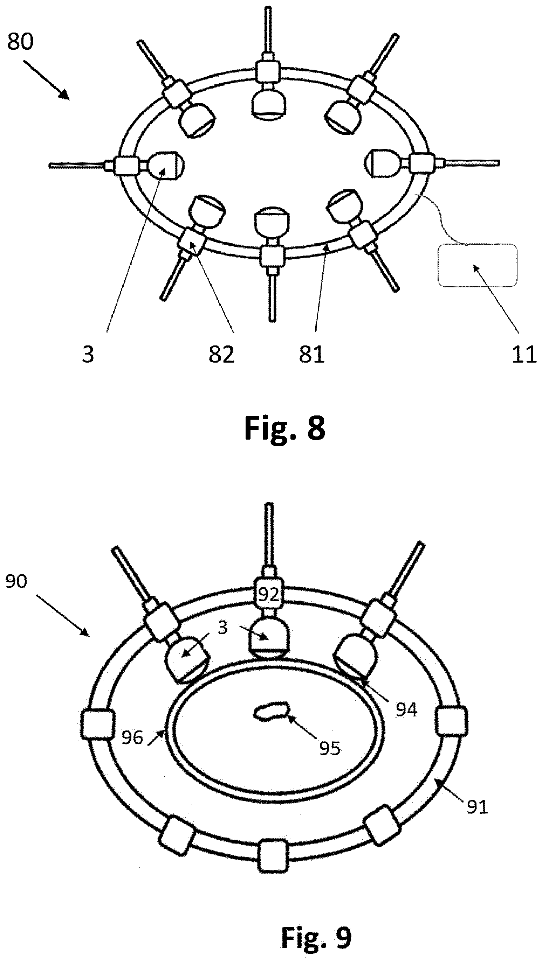

Bei mehreren Applikatoren (siehe Figuren 8, 9) ist die Überlagerung von Stoßwellen aus verschiedenen Richtungen möglich, wodurch die i.d.R. auch lokal in verschiedenen Richtungen ausgeprägten Amyloid-Strukturen wirksam behandelt werden.

Die Steuervorrichtung 11 kann eine Frequenzsteuereinheit, eine Impulssteuereinheit, eine Impulsfrequenzsteuereinheit, eine Fokussteuereinheit und/oder eine Steueranordnung umfassen, wobei die Steueranordnung dazu ausgelegt ist, einen Betriebsmodus zur Behandlung von Alzheimererkrankungen einzunehmen.

Figur 2 zeigt eine schematische Darstellung eines zweiten Beispiels für eine erfindungsgemäße Vorrichtung 20, wobei eine Ortung der Vorrichtung schematisch dargestellt ist.

Diese umfasst Patienten DICOM Daten 21, Rigid Body Modell 22 des Patientenkopfes, eine optische Ortung 23 (z.B. eines NDI Polaris Systems), Rigid Body SW Applikator 24, d.h. ein Modell eines Therapiekopf eines Applikators und dessen Position und Ausrichtung im Raum, einen Stoßwellen-Applikator 3, DICOM-MATLAB-FEM Simulationsmodel 26 des Patientenschädels, d.h. ein Simulationsmodell des Kopfes auf Basis der gemessenen Daten, welches in Übereinstimmung gebracht wird mit der Position des Patientenkopfes, der SW Propagation in das AD Areal, DICOM-MATLAB-TABLEIN-FEM Übertragung 27 zur Positionierung bzw. Kontrolle der Position des Therapiekopfes am Patienten sowie der genauen Ausrichtung auf die zu behandelnden AD-Areale.

Ein Patientenkopfes wird auf Basis von Patientendaten sowie dessen Position und Ausrichtung im Raum repräsentiert, wobei der Patient z.B. eine Brille mit Markern trägt, die von der optischen Ortung erfasst werden.

Aus den Patienten DICOM Daten 21 wird ein Rigid Body Modell 22 des Patientenkopfes erstellt. Es wird sodann ein DICOM/MATLAB/FEM-Simulationsmodell 26 des Schädels erstellt, bei dem ein Rigid Body Modell-Stoßwellenapplikator 24 Stoßwellen auf diesen appliziert und die Stoßwellen-Propagation in das AD Areal berechnet. Aus der DICOM/MATLAB/FEM-Simulation folgen dann die Behandlungsparameter, bzw. die Einstellparameter.

Wie oben beschrieben, sind DICOM, MATLAB und FEM stellvertretende Komponenten der Berechnung, die stellvertretend für ähnliche Systeme, die (auch in Kombinationen mit einigen der genannten Komponenten) annähernd die gleichen bzw. analoge Simulationen bzw. Berechnungen ausführen können. Solche Systeme werden als DICOM/MATLAB/FEM-Programmsystem subsummiert.

Der Patient setzt eine Brille mit Markern auf, mit der über das Ortungssystem seine Kopfposition und Ausrichtung erfasst werden. Ebenfalls wird auf dem Applikator bzw. Therapiekopf ein Marker appliziert, so dass das Ortungssystem die genaue Position und Ausrichtung des Therapiekopfes in Relation zum Patientenkopf erfasst bzw. berechnet.

Die Applizierung der Stoßwellen am Patienten kann sodann vorgenommen werden, wobei die optische Ortung 23 (z.B. NDI Polaris System) dabei verwendet wird, die Position und Richtung des realen Stoßwellenapplikators 3 am realen Schädel des Patienten (entsprechend der Überlagerung mit dem Schädelmodell 26) richtig zu positionieren. Dabei müssen die Koordinaten des Simulationsmodell des Patientenkopfes mit den Koordinaten des realen Kopfes in Übereinstimmung gebracht werden.

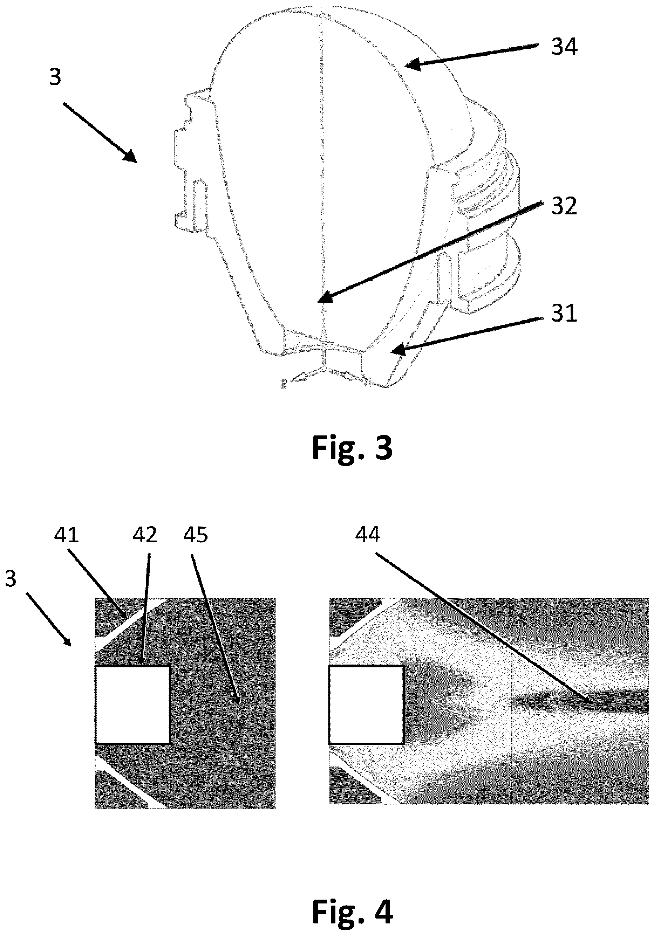

Fig. 3 enthält eine schematische 3D Darstellung eines Beispiels für einen fokussierenden Applikator 3 einer elektrohydraulischen Stoßwellenvorrichtung 10 (siehe Figur 1) mit folgenden Komponenten: Reflektor 31, Übertragungsmedium 32, Membran 34.

In den Reflektor 31 (z.B. mit der Form eines Ellipsoids, eines Paraboloids, eines Kegel- oder Kegelstumpfes oder einer Freiformfläche) wird im unteren Teil die Elektroden (nicht gezeigt) positioniert und fixiert. Die Elektroden liegen i.d.R. auf der Mittelachse. Der Spalt zwischen den Elektroden liegt z.B. im Fokuspunkt bzw. bei geringerer Fokussierung auch außerhalb des Fokuspunktes. Als Übertragungsmedium 32 wird i.d.R. Wasser (ggf. mit Zusätzen) verwendet. Während der Entladung entsteht ein Plasma-Kanal, der zu einer explosionsartigen Ausdehnung einer Gasblase führt, welche die Stoßwelle erzeugt. Bei elektrohydraulischen Systemen bewegt sich die anfängliche Stoßfront der Stoßwelle dabei mit einer Geschwindigkeit, die höher liegt als die Schallgeschwindigkeit des Wassers. Die Stoßwellen breiten sich im Reflektor aus, werden an dessen inneren Oberflächen reflektiert und propagieren zur Membran 34, die i.d.R. mit einem Gel direkt am Schädel des Patienten anliegt (nicht im Bild dargestellt).

In Fig. 4 sind weitere Darstellungen eines Applikators 3 gezeigt. Der Applikator 3 gehört zu einer elektromagnetischen Stoßwellenvorrichtung und weist folgende Komponenten auf: Reflektor 41, elektromagnetische Spule 42, Übertragungsmedium 45; Auf der rechten Seite ist die Verteilung der Druckfelder der elektromagnetischen Stoßwellenpropagation zu sehen. Deutlich ist die starke Fokussierung 44 zu erkennen.

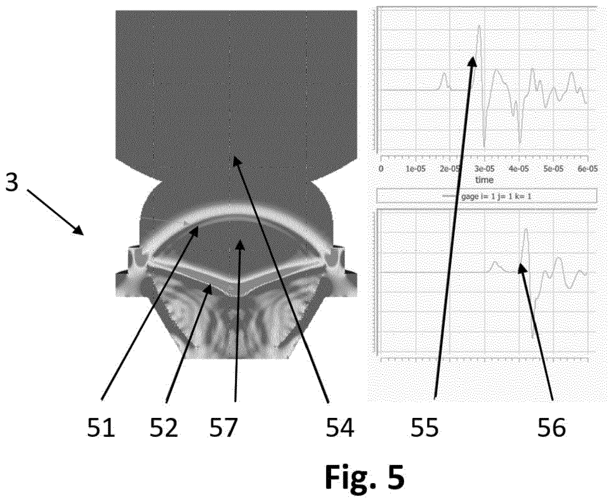

Figur 5 zeigt eine weitere Darstellung eines Applikators 3 und zugehörige Druckkurven. In Fig. 5 ist zudem die simulierte Propagation durch die Schädeldecke für einen elektrohydraulischen Stoßwellengenerator dargestellt. Zu sehen ist die primäre radiale Druckwelle 51, die reflektierte Druckwelle 52, Auswertungspunkt a) 57, Auswertungspunkt b) 54. Auf der rechten Seite ist der Druckverlauf 55 im Auswertungspunkt a) und der Druckverlauf 56 im Auswertungspunkt b) dargestellt.

Die Stoßwelle hat einen scharfen positiven Peak, gefolgt von einer negativen Druckanteilen.

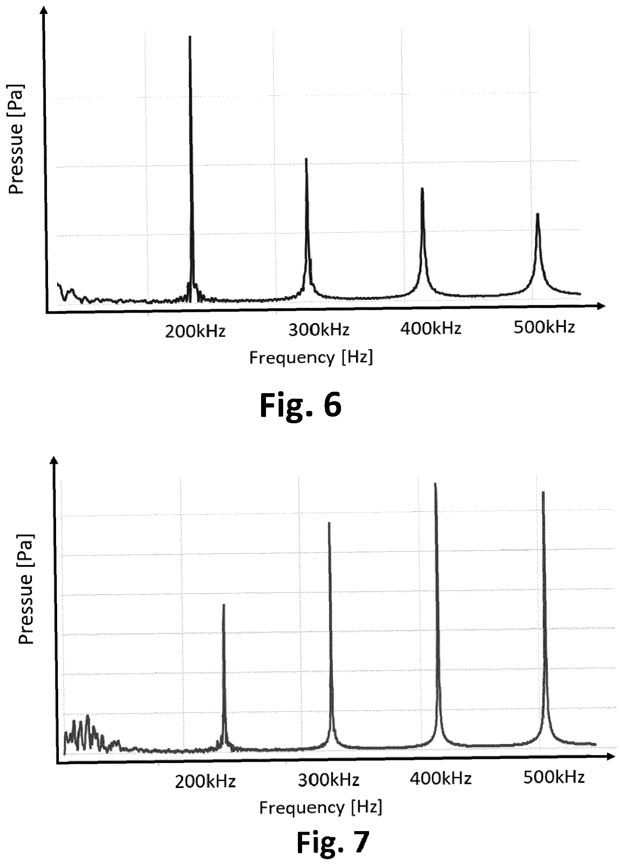

Figur 6 zeigt ein erstes Beispiel für ein Frequenzspektrum eines Druckverlaufs.

Es ist die beispielhafte Verteilung der Frequenzbänder im FFT Spektrum eines Druckverlaufs einer Stoßwelle bei einer angenommenen Entladungsfrequenz des Entladungsschwingkreises von 200 kHz dargestellt (zum besseren Verständnis als Schmalbandspektrum dargestellt). Hierbei wird angenommen, dass die Entladefrequenz sich auch im Drucksignal analog wiederfindet. Dies kann u.U. variieren, daher ist diese Betrachtung hier nur schematischer Natur. Peaks unterhalb von 200 kHz sind hier nicht dargestellt. Bei ca. 200 KHz ist ein ausgeprägtes Maximum zu erkennen. Deutlich sind Maxima bei höheren Frequenzen zu erkennen. Frequenzbänder des Druckverlaufes unterhalb von 450 kHz sind günstig, um durch den Schädel zu propagieren, der schwingungstechnisch wie ein Tiefpassfilter wirkt.

Grundsätzlich ist es für die Alzheimerbehandlung wichtig, dass möglichst hochfrequente Druckwellen in den Schädel gelangen. Dies ist jedoch aufgrund der beschriebenen TiefpassCharakteristik des Schädels nur bis zu einer bestimmten Frequenz, z.B. 450kHz möglich. Druckwellenanteile oberhalb dieser Frequenz werden nicht übertragen bzw. stark gedämpft. Desto tiefer die Grundfrequenz der Druckwellen ist, desto mehr hochfrequente Anteile (hier als Harmonische im Schmalbandspektrum dargestellt) können in den Schädel propagieren.

Figur 7 zeigt ein zweites Beispiel für ein Frequenzspektrum eines Druckverlaufs.

Es ist die beispielhafte Verteilung der Frequenzbänder im FFT Spektrum eines Druckverlaufs einer Stoßwelle bei einer angenommenen Entladungsfrequenz des Entladungsschwingkreises von 450 kHz dargestellt. Weitere Annahmen sind wie oben beschrieben. Hier ist auch ein niedriges Maximum bei 450kHz und weitere Maxima bei höheren Frequenzen zu erkennen. Hier wird die meiste Energie in den Frequenzbändern oberhalb von 450kHz übertragen (hier als Harmonische im Schmalbandspektrum dargestellt). Dies ist für die Durchdringung des Schädels nicht optimal, da der Schädel eine Tiefpasscharakteristik besitzt und oberhalb von 450kHz praktisch keine Druckwellen in den Schädel gelangen bzw. stark gedämpft werden.

Figur 8 zeigt eine schematische Darstellung eines dritten Beispiels für eine erfindungsgemäße Vorrichtung 80. Es ist die Anordnung der Vorrichtung 80 mit mehreren SW Applikatoren 3 dargestellt. Zu sehen sind der SW Applikator 3 mit Übertragungsmedium, Positionierung 82 der SW-Applikatoren auf einem Positionierungsring 81. Anstelle eines Positionierungsrings 81 können die Applikatoren auch auf einem Halbring, Oval, oder anderen stangenförmigen Positionierungsreinrichtungen (z.B. auch in Kugel- oder Halbkugelform) montiert werden. Mit der Steuervorrichtung 11, die eine Modulation/ Steuerung umfasst, werden die SW Applikatoren 3 angesteuert. Dadurch lassen sich die Stoßwellen bei einem Schädel (nicht gezeigt) gezielt aus verschiedenen Richtungen applizieren.

Figur 9 zeigt eine schematische Darstellung eines vierten Beispiels für eine erfindungsgemäße Vorrichtung 90. Die Vorrichtung 90 ist analog zu der Vorrichtung 80 aus Figur 8, hier nur mit drei SW Applikatoren 3, die an dem Positionierungsring 91 des Positionierungsmechanismus 92 befestigt sind und am Schädelknochen 91 über Übertragungsgel 94 an den Schädel 91 angekoppelt sind. Die SW Applikatoren 3 applizieren Stoßwellen aus drei Richtungen auf AD Areal 95.

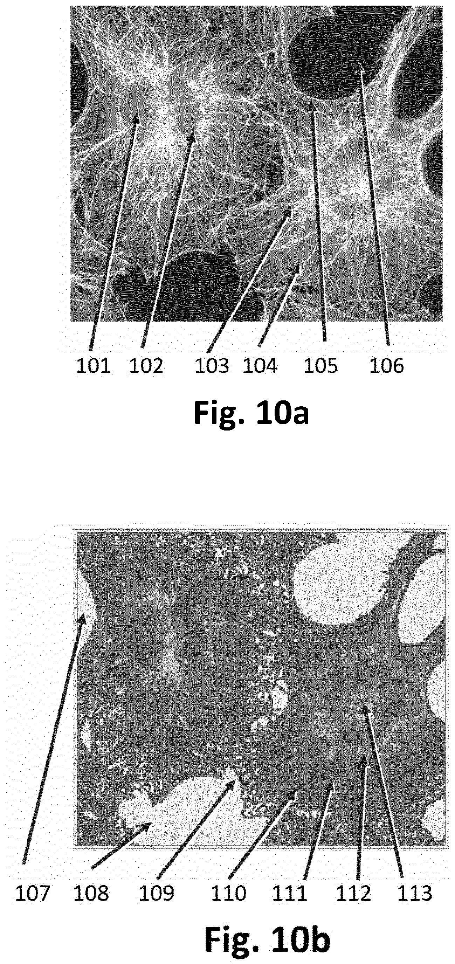

Figuren 10a und 10b zeigen zelluläre Strukturen. In Fig. 10a, 10b sind die Ergebnisse der FEM Simulation der Stoßwellenpropagation und deren Wirkung auf patientenindividuelle zelluläre Strukturen dargestellt. In Fig. 10a zu sehen sind die Kernmembran 102, Mikrofilamente 103, Aktinfilamente 104, Zellmembran 105, Zellkern 101, Extrazelluläre Matrix (ECM) 106, wie sie von einem bildgebenden Verfahren geliefert werden. In Fig. 10b ist die MATLAB Übertragung der patientenindividuellen Zellstrukturen auf ein nichtlineares FEM Model der Stoßwellenpropagation zu sehen: 107, dabei sind: ECM (extrazelluläre Matrix) 108, Zellmembran 109, Aktinfilamente 110, Mikrofilamente 111, Kernmembran 112 und der Zellkern 113. Es ist im Vergleich von Fig. 10a and Fig. 10b deutlich zu sehen, dass die zellulären Strukturen entsprechend der Auflösung der 3D-Bildaufnahmesysteme mit einer sehr hohen Auflösung in Fig. 10b nachgebildet werden. Hierbei werden den Strukturen lokal unterschiedliche Materialparameter zugewiesen. In der Simulation durchdringt die Stoßwelle die Zellareale und die Wirkung auf unterschiedliche Zellstrukturen kann durch einen Farbumschlag (z.B. zur Darstellung von Dehnung oder ggf. auch der Spannung in unterschiedlichen Farben) erkennbar gemacht werden.

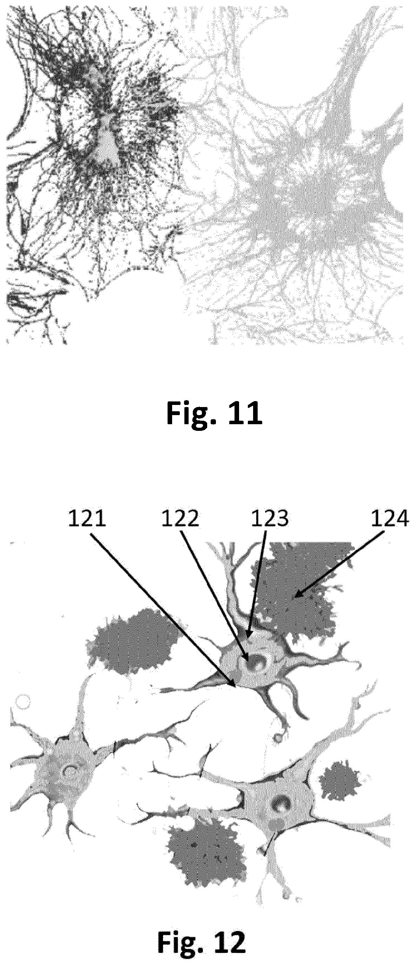

Figur 11 zeigt eine Darstellung eines Ergebnisses einer FEM-Simulation. In Fig. 11 ist beispielhaft das Ergebnis der FEM-Simulation der Stoßwellenpropagation durch eine Zellstrukturen dargestellt. Die Abbildung enthält die Dehnungsfelder zu einem bestimmten Zeitpunkt. Man erkennt die Stoßwellenfront, die in der Mitte von Fig. 11 vertikal verläuft.

In Fig. 12 sind die zelluläre Strukturen krankhafter Proteinablagerungen in AD-Arealen dargestellt. Zu sehen sind Tau-Fibrillen 121, der Nukleus (Zellkern) 122, das Zytoplasma 123 und die Amyloid Proteinablagerungen 124. Die Abmessungen und die physikalischen Eigenschaften werden über die Programme MATLAB, PZFLEX, ANSYS auf ein FEM Simulationsmodell übertragen und numerisch gelöst. Es resultieren die Behandlungsparameter für die Fragmentation und Auflösung der Amyloid Ablagerungen.

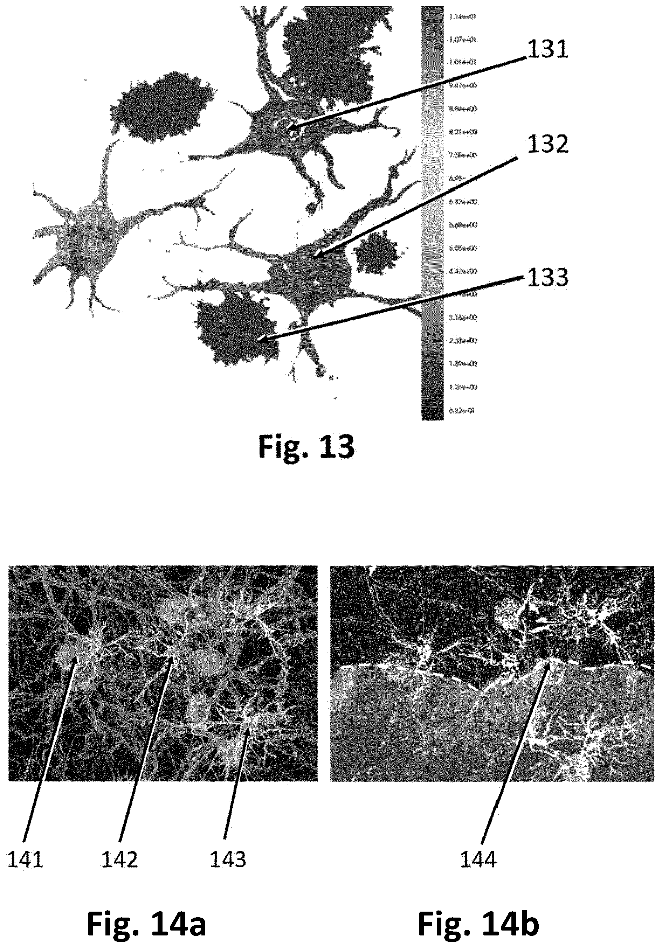

Figur 13 zeigt eine beispielhafte Darstellung von Dehnungsfelder in zellulären Strukturen. Die Intensität der Graustufe ist ein Maß für die Dehnung. In Fig. 13 sind letale Dehnungsfelder in Amyloid Proteinablagerungen 133 dargestellt. Gesunde zerebrale Areale (Nukleus 131, Zytoplasma 132) bleiben annähernd unbelastet, da sie aufgrund ihrer geringeren Steifigkeit die Dehnungen unbeschadet überstehen.

Figuren 14a und 14b zeigen Darstellungen von FEM-Simulationsanalysen. In Fig.14a/b sind patientenindividuelle FEM-Simulationsanalysen der nichtlinearen Stoßwellenpropagation durch das Gehirnareal dargestellt. In Fig. 14a sind die Amyloid-Ablagerungen 141, die Neuronen 142, Tau-Fibrillen 143 zu sehen. In Fig. 14b dargestellt ist die Propagation der Druckfront 144 auf zellulärer Ebene.

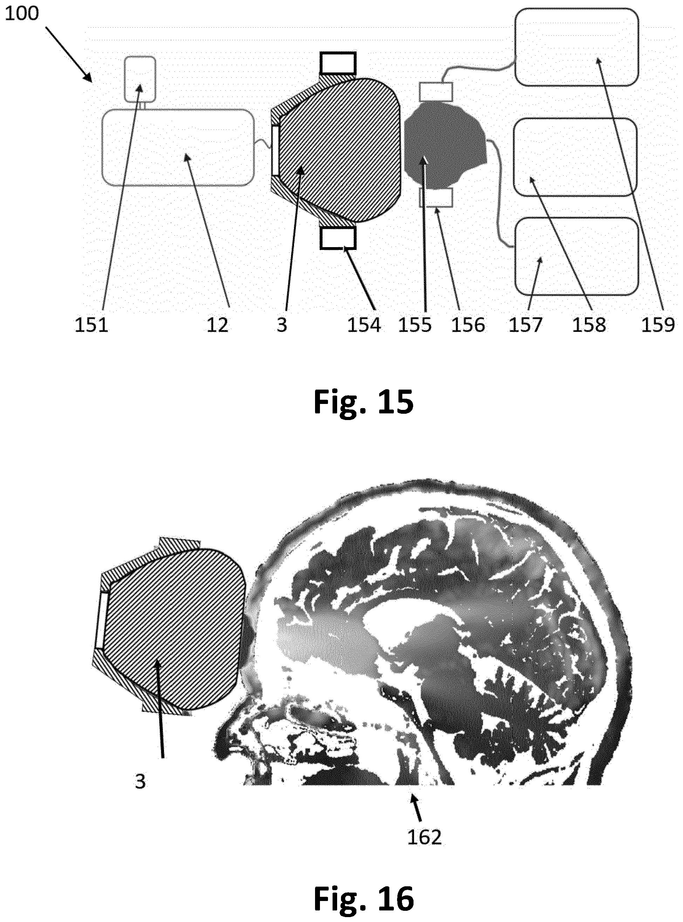

Figur 15 zeigt ein Beispiel für System. In Fig. 15 sind die Komponenten Systems 100 für die extrakorporale Behandlung von Alzheimer Erkrankungen mit Hilfe alternierender mechanischer Felder (AMF), alternierender elektrischer Felder (ETF TTF) und Singlet Sauerstoff (H3) dargestellt. Zu sehen sind die Steuervorrichtung 151 der (AMF) kapazitiven Entladung, der Stoßwellengenerator 12, die Stoßwellen-Applikatoren 3 mit Übertragungsmedium (Vorrichtung mit zwei Applikatoren nicht dargestellt), der Positionierungsmechanismus 154 des Stoßwellenapplikators, das Alzheimer Areal 155 (schematisch, Schädel nicht dargestellt), die TTF Elektroden 156, eine Temperiervorrichtung 157 für die Temperierung des Alzheimer Areales, die H3-Zufuhr-Vorrichtung 158 und die TTF Vorrichtung 159.

Figur 16 zeigt ein erstes Beispiel für eine simulierte Stoßwelle. Fig. 16 zeigt die Darstellung eines simulierten SW-Applikators 3 (mit Gummimembran), Übertragungsgel, Gehirnschnittbild, hellere Bereiche zeigen die simulierte Druckverteilung einer Stoßwelle die durch das Gehirn propagiert. Es ist zu sehen, dass hier bei einer wenig bis nicht fokussierten Stoßwelle eine gleichmäßige Druckbeaufschlagung über weite Bereiche des Gehirns erzielt wird.

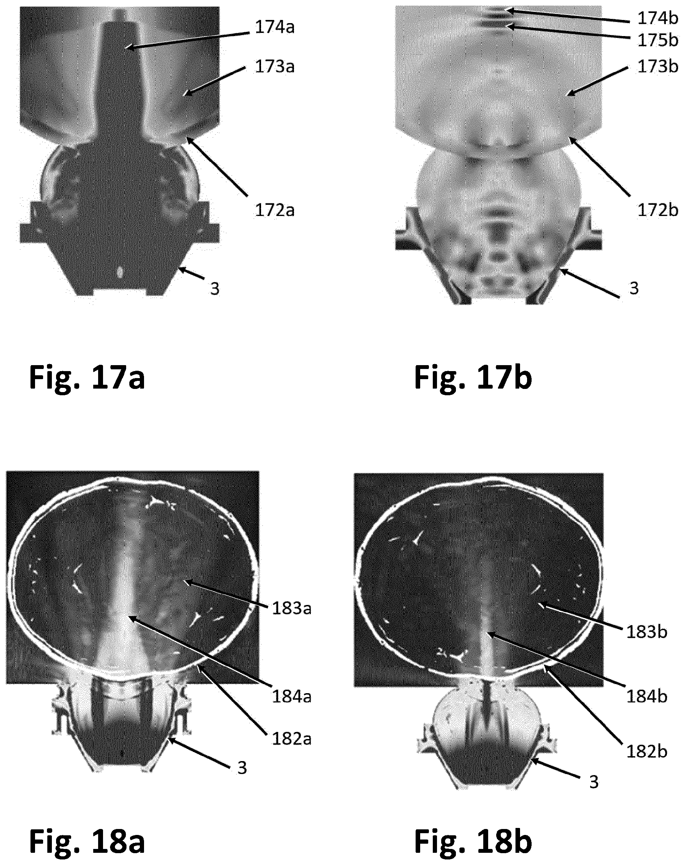

Figuren 17a und 17b zeigen weitere Beispiele für ein simulierte Stoßwellen. Fig. 17a zeigt die Simulation der Richtwirkung eines schwach bzw. unfokussierten SW-Applikators 3 durch einen Schädel 172a in das Gehirn 173a. Der Behandlungsbereich 174a (Maximalwerte bei einer behandlungstypischen Druckamplitude) ist deutlich zu erkennen. Fig. 17b zeigt Simulation der Richtwirkung eines schwach bzw. unfokussierten SW-Applikators 3 durch einen Schädel 172b in das Gehirn 173b. Zu sehen ist hier eine Sequenz des zeitliche Druckverlauf der Stoßwellenpropagation. Deutlich ist am oberen Rand des Bildes die erste Druckwelle 174b gefolgt von einer breiten zweiten Druckwelle 175b mit erhöhter Amplitude zu sehen.

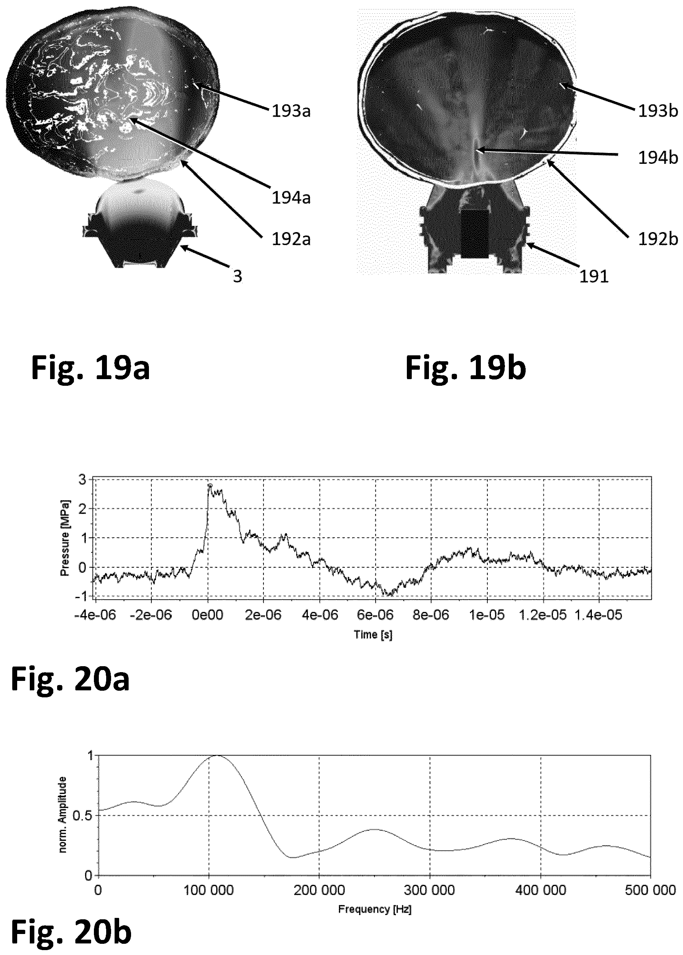

Figuren 18a und 18b zeigen weitere Beispiele für ein simulierte Stoßwellen. Fig. 18a/b zeigen den Unterschied zwischen einer Gehirnbehandlung mit einer schwach bzw. unfokussierten Stoßwelle und einer fokussierten Stoßwelle (Simulationen). Fig. 18a: bei der schwach bzw. unfokussierten Stoßwelle des simulierten SW Applikators 3 wird durch den Schädel 182a ein weiter Bereich des Gehirnes 183a mit der Stoßwelle und niedriger Druckamplitude schonend behandelt. Hell ist hier der Behandlungsbereich 184a dargestellt. In Fig. 18b ist zu sehen, dass bei der fokussierten Stoßwelle des simulierten SW Applikators 3 bei der Propagation durch den Schädel 182b nur ein kleiner Bereich des Gehirnes 183b mit der Stoßwelle behandelt wird. Der Behandlungsbereich 184b ist hier sehr viel kleiner als im Bild 18a. Es ist davon auszugehen, dass auch der Schädel 182b aufgrund seiner Krümmung eine zusätzlich fokussierende Wirkung hat. Eine solche doppelte Fokussierung kann bei fokussierten Stoßwellen u.U. zu hohen Spitzendrücken und u.U. zu Zell- und Gewebeschädigungen führen. Figuren 19a und 19b zeigen weitere Beispiele für ein simulierte Stoßwellen. Fig. 19a/b zeigen den Unterschied zwischen einer Gehirnbehandlung mit einer unfokussierten Stoßwelle eines elektrohydraulischen SW-Applikators 3 und einer fokussierten Stoßwelle eines elektromagnetischen SW-Applikators 191 in der Simulation. Fig. 19a: die unfokussierte Stoßwelle des SW Applikators 3 durchdringt den Schädel 192a und ein weiter Bereich 194a des Gehirns 193a wird somit behandelt. Fig. 18b: die fokussierte Stoßwelle des piezoelektrischen SW Applikators 191 durchdringt den Schädel 192b und nur ein sehr kleiner Bereich 194b des Gehirns 193b ist behandelbar.

Figuren 20a und 20b zeigen einen Druckverlauf einer Stoßwelle und das zugehörige Frequenzspektrum. Fig. 20a zeigt den zeitlicher Druckverlauf eines Applikators, der mit einem 10m langen Applikatorkabel versehen ist, d.h. die Induktivität zwischen den Elektroden und dem Kondensator wurde deutlich erhöht. Es ist zu sehen, dass der zeitliche Anstieg der Stoßwelle relativ langsam erfolgt. In Fig. 20b zeigt die FFT Analyse (normierte Amplitude) des zeitlichen Druckverlaufs mehrere dominante Frequenzbänder bzw. -bereiche im Bereich von ca. 90kHz bis 450kHz.

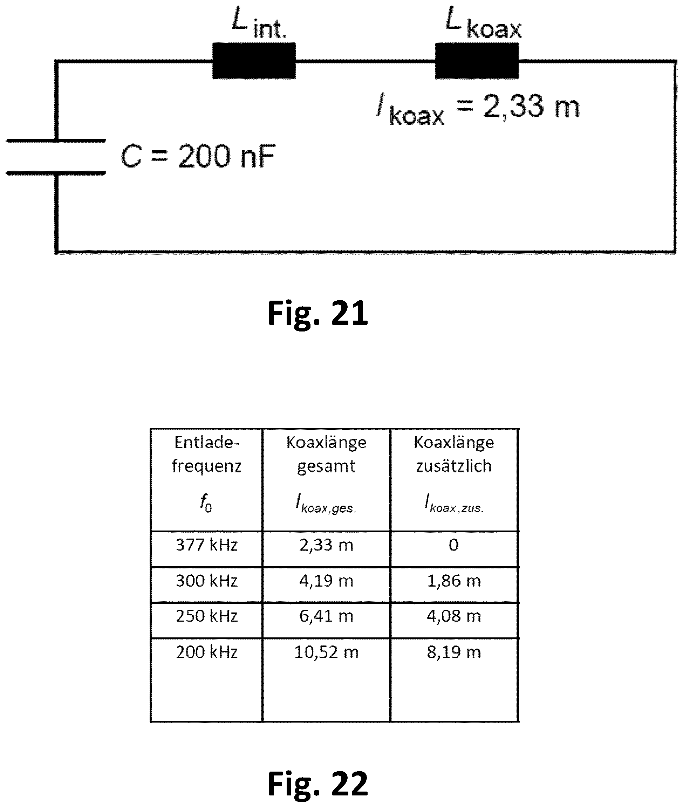

In Fig. 21 ist das Ersatzschaltbild eines Schwingkreises bestehend aus dem Kondensator des Stoßwellengeräts mit der internen Induktivität Lᵢₙₜ sowie der Induktivität des koaxialen Hochvoltkabels Lₖₒₐₓ im Moment der Entladung (Elektroden kurzgeschlossen) dargestellt. Die Zahlenwerte dienen nur der Orientierung. Bei gegebener Entladefrequenz (Messung) bekannter Stoßkapazität und interner Induktivität kann die erforderliche Induktivität des koaxialen Hochvoltkabels berechnet mit der Gleichung ω= 1/sqrt (C (Lᵢₙₜ+Lₖₒₐₓ)) und der Auflösung nach Lₖₒₐₓ berechnet werden. Bei gegebenen, längenbezogenen Induktivätsbelag L_{koax'} (typischer Wert z.B. 278 nH/m) kann die Länge lₖₒₐₓ des koaxialen Hochvoltkabels berechnet werden zu: lₖₒₐₓ = Lₖₒₐₓ / L_{koax'}. Um die Länge der koaxialen Hochvoltkabel kurz zu halten, können auch höherinduktive koaxiale Hochvoltkabel verwendet werden. Im Stand der Technik gibt es weitere Möglichkeiten, um die Induktivität eines koaxialen Hochvoltkabels zu reduzieren bzw. zu erhöhen. Dieser Stand der Technik kann ebenfalls zur Induktivitätserhöhung der koaxialen Hochvoltkabel herangezogen werden und kürzere Längen des koaxialen Hochvoltkabels ermöglichen.

Es sei angemerkt, dass auch hohe Entladefrequenzen des Stoßkondensators vorteilhaft sein können, z.B. um die Leistung zu erhöhen und stärkere Stoßwellen zu generieren. Hierzu kann die Induktivität des koaxialen Hochvoltkabel bzw. die Induktivität zwischen Stoßkondensator und Applikator vermindert werden. Dazu kann beispielsweise die Länge des koaxialen Hochvoltkabels verringert werden oder es können anstelle eines koaxialen Hochvoltkabels mehrere (dünnere) koaxiale Hochvoltkabel parallelgeschaltet werden.

Fig. 22 zeigt einen beispielhaften Zusammenhang zwischen den Kondensator-Entladefrequenzen und der Applikatorkabellänge. Die Kabellängen können durch die Verwendung von höheren Induktivitätsbelegen (L_{koax'} > 278nH/m) verkürzt werden.

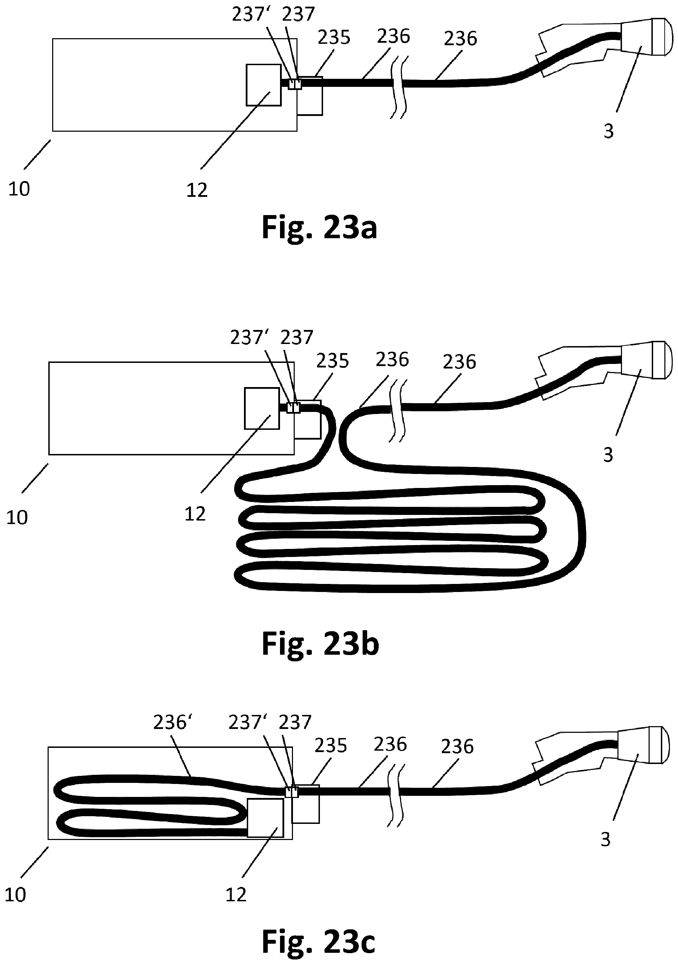

Figuren 23a-23g zeigen Beispiele für Stoßwellengeräte 10 mit unterschiedlichen Induktivitäten. Hier ist stets, zum Beispiel in Fig. 23a, ein Stoßwellengerät 10 mit Stoßwellengenerator 12 zu sehen. Es sind in den Figuren nur diejenigen Bauteile dargestellt, die für die Erläuterungen wichtig sind. Der Applikator 3 mit Reflektor ist über ein koaxiales Hochvoltkabel 236 und Verbindungselemente 235, 237, 237' (Stecker, Buchse o.ä.) mit dem Stoßwellengenerator 12 verbunden. Man verwendet grundsätzlich koaxiale Hochvoltkabel, da diese elektromagnetisch abgeschirmt sind. Aufgrund der hierzu notwendigen Isolation zwischen stromführenden Innenleiter und der äußeren Abschirmung ergeben sich die erforderlichen, relativ großen Biegeradien sowie eine hohe Biegesteifigkeit des axialen Hochvoltkabels. Der stromführende Innenleiter ist in den Figuren nicht explizit dargestellt.

In Fig. 23a ist der Ausgangszustand mit einem kurzen koaxialen Hochvoltkabel 236 dargestellt, welches i.d.R. kleiner gleich 2m lang ist. Als zusätzliche Induktivität wird nun in Fig. 23b ein sehr viel längeres koaxiales Hochvoltkabel 236 verwendet. Die Länge des koaxialen Hochvoltkabel 236 ist deutlich länger als 2m, vorzugsweise länger als 5m und noch vorzugsweiser zwischen 9 und 15 Metern lang. Das koaxiale Hochvoltkabel 236 kann auch länger als 15 Meter sein. Eine lange Verbindung zwischen dem Stoßwellengenerator 12 und dem Applikator 3 ist in vielen Behandlungssituationen durchaus vorteilhaft. Somit muss das Stoßwellengerät 10 mit dem Stoßwellengenerator 12 dann nicht versetzt oder verschoben werden, da man den Applikator 3 in einem weiten Umkreis vom Stoßwellengerät verwenden kann.

In Fig. 23c ist ein Teil des koaxialen Hochvoltkabels 236` im Stoßwellengerät 10 untergebracht und ist dann am Ende mit dem Stoßwellengerator 12 verbunden. Die Länge des koaxialen Hochvoltkabels 236` im Stoßwellengerät beträgt mehr als 1m, vorzugsweise mehr als 2m, noch vorzugsweiser mehr als 5m. Hier ist bei der Verlegung des koaxialen Hochvoltkabels 236` im Stoßwellengerät 10 ebenfalls auf die minimal zulässigen Biegeradien des koaxialen Hochvoltkabels 236` zu achten. Gegebenenfalls kann das koaxiale Hochvoltkabel 236` noch mehrmals unterteilt werden und die Stücke über weitere Verbindungselemente verbunden sind. Durch diese Maßnahme kann den Platzbedarf für das koaxiale Hochvoltkabel 236` drastisch reduziert werden.

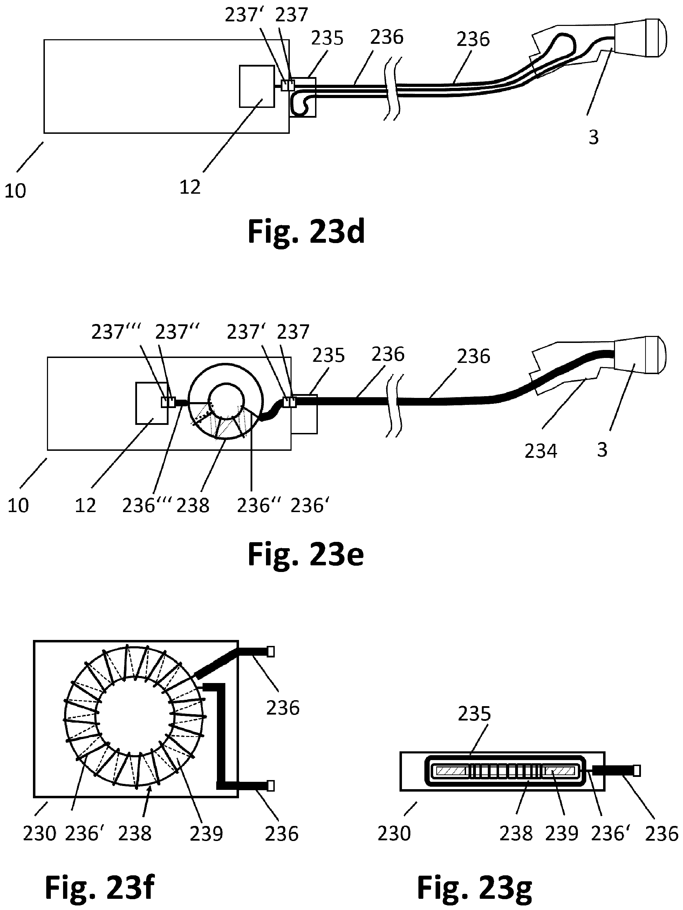

In Fig. 23d wird ein langes, dünnes koaxiales Hochvoltkabel 236 verwendet, welches zwischen dem Applikator 3 und dem Stoßwellengenerator 12 in Schleifen hin und her und wieder hinläuft, d.h. es laufen dann z.B. drei koaxiale Hochvoltkabelstränge nebeneinander. Falls erforderlich kann das koaxiale Hochvoltkabel auch noch weitere Male zwischen Applikator 3 und Stoßwellengerät 10 her und hin laufen. Zur Berücksichtigung der Biegeradien, wird das koaxiale Hochvoltkabel 236 teils im Stecker 237, 237` oder im Verbindungselement 235 bzw. teils im Applikator 3 umgebogen und untergebracht, um dann in der entgegengesetzten Richtung weiterzulaufen. Anstatt über Biegung mit großem Biegeradius können die Kabel auch platzsparend über Schraubverbindungen z.B. U-förmig umgelenkt werden. Die Verbindungen sind jedoch gesondert abzuschirmen.

Dünnere koaxiale Hochvoltkabel lassen sich z.B. dadurch realisieren, dass die Spannung für die elektrohydraulische Stoßwellengenerierung z.B. durch die Verwendung besonders leitfähiger Fluide (Wasser mit Zusätzen), geringeren Elektrodenabstand oder auch eine höhere Stoßwellenwiederholraten signifikant herabgesetzt wird.

In Fig. 23e wird zur Realisierung einer zusätzlichen Induktivität zwischen dem Applikator 3 und dem Stoßwellengenerator 12 eine Toroid- bzw. Ringkernspule verwendet, bei der der stromführende Leiter des koaxialen Hochvoltkabels einmal oder mehrfach durch das Innere der Ringkernspule geführt wird. Die Induktivität des stromführenden Leiters mit der Ringkernspule steigt quadratisch mit der Anzahl der Wicklungen. Wenn anstelle der oder zusätzlich zur der Ringkernspule ein Klappferrit verwendet wird, so kann damit der Stromanstiegs bis zum Sättigungsstrom verzögert werden, wodurch sich auch das Frequenzspektrum der Stoßwellendruckverlaufs ebenfalls zu tieferen Frequenzen verschieben kann.

In Fig. 23f wird gezeigt, wie anstelle eines langes Hochvoltkabels eine hohe Induktivität auf engem Raum untergebracht wird. Dies gelingt dadurch, dass in eine flachen Kassette 230, die Teil des Stoßwellengerätes 10 (siehe vorhergehende Figuren) ist oder unter bzw. an dem Stoßwellengerät angebracht ist. Der Innenleiter 236` des koaxialen Hochvoltkabels 236 ist spiralförmig verlegt, so dass hierdurch eine Toroid-Spule 238 resultiert, die zum Beispiel horizontal in der Kassette 230 angeordnet ist.

Je nach Größe kann die Toroid-Spule auch anderwertig in dem Stoßwellengerät untergebracht werden. Die Toroid-Spule 238 trägt ebenfalls zu einer Erhöhung der Induktivität des verlegten Hochvoltkabels bei. Die Anzahl der Windungen geht quadratisch und die Fläche des Kerns 239 sowie der mittlere Radius des Kerns 239 gehen linear in die Induktivität ein. Bei einem Kern aus Luft kann die Anzahl der Windungen oberhalb von 30, vorzugsweise oberhalb von 50 und weiter bevorzugt oberhalb von 100 liegen, um hohe Induktivitäten zu realisieren.

Im Innern der Toroid-Spule 238 kann sich Luft, Kunststoff sowie zusätzlich oder anstelle dessen ein Ferrit- oder Eisenpulverkern befinden. Dadurch kann die Induktivität weiter erhöht werden, bzw. die Windungsanzahl reduziert werden.

Wird ein Kern bestehend aus Luft (oder Kunststoff) und einem Ferrit- oder Eisenpulverkern verwendet, so kann der Ferrit- oder Eisenpulverkern so ausgelegt werden, dass er groß genug ist, um nicht in die Sättigung zu gehen.

Zur elektromagnetischen Abschirmung kann die Spule mit aus dem Stand der Technik bekannten Mitteln versehen sein (in diesem Bild nicht gezeigt), zum Beispiel einen Käfig 235 (siehe Figur 23g). In einem Stoßwellengerät 10 können auch mehrere Kassetten 230 verwendet werden oder mehrere Spulen 238 in eine Kassette 230.

In Figur 23g ist die Kassette 230 aus Figur 23f in der Schnittdarstellung von der oberen Seite zu sehen. In dieser Ansicht ist nur ein Anschluss 236 zu sehen (der andere Anschluss ist nicht zu sehen. Die Toroid-Spule 238 kann als elektromagnetische Abschirmung einen Käfig 235 besitzen, z.B. realisierbar als Drahtgeflecht, Gitter, Stahlplatte, insbesondere umfassend magnetischen Materialien.

Die Abschirmung kann auch an der Oberfläche der Kassette 230 angebracht sein und/oder die Wände der Kassette 230 können abschirmendes Material umfassen.

Der Kern 239 kann wiederum aus Luft oder den vorab genannten Materialen bestehen. Grundsätzlich kann bei den im Patent erwähnten Toroidspulen mit einem magnetisch leitenden ringförmigen Kern auch ein Spalt im Kern vorgesehen werden, um eine Sättigung der Spule zu vermeiden.

Die Kassettenlösung ermöglicht die Auf- bzw. Nachrüstung eines bestehenden Gerätes für die Stoßwellengenerierung in niedrigen Frequenzbändern. Hierzu wird die Kassette über elektrischen Leitungen 236 vor das stromzuführende Hochvoltkabel des Applikators 3 in Serie geschaltet.

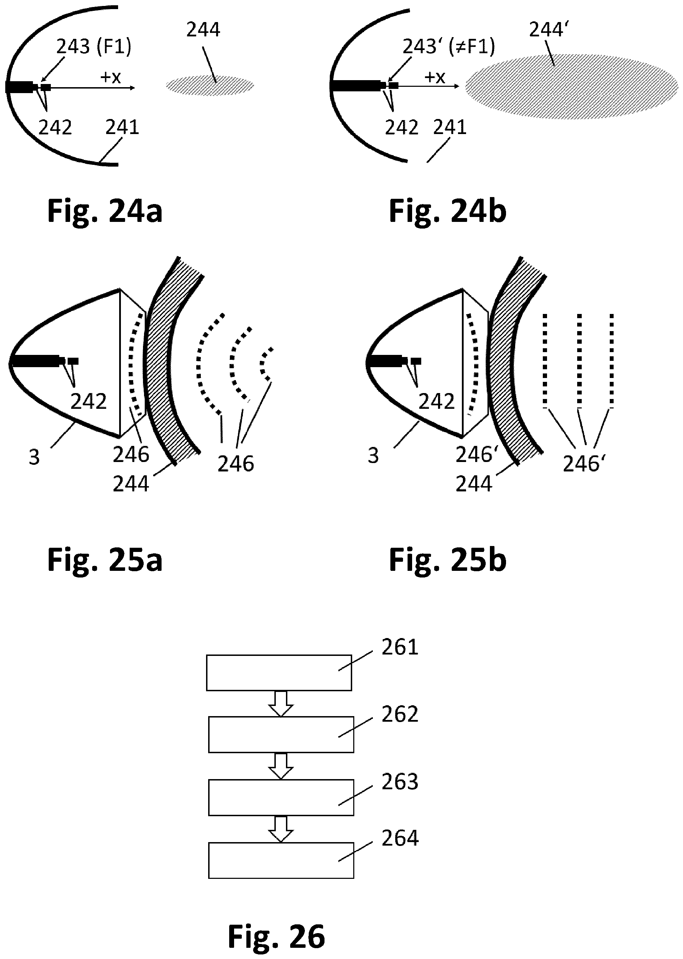

Figuren 24a und 24b zeigen Reflektoren 241 mit Elektroden 242 in zwei verschiedenen Positionen.

Es wird veranschaulicht, dass eine Änderung der Elektrodenposition in +x-Richtung bzw. in Richtung auf das Behandlungsgebiet eine Verbreiterung des Fokus zur Folge hat. In Fig. 24a ist ein Reflektor 241 mit Elektroden 242 zu sehen. Die Mitte der Elektroden 242 ist etwa axial im Fokus-Punkt 243 des Ellipsoids oder des Paraboloids gelegen. In Fig. 24b ist der Reflektor 241 axial kürzer gestaltet, idealerweise ist die axiale Länge auf der Innenseite kleiner gleich 35mm. Die Lage der beiden Elektroden 242` ist idealerweise an einem Punkt 243', der nicht der Fokus-punkt ist. Der Abstand der Elektrodenmitte zum Fokuspunkt beträgt 3-15mm, vorzugweise 3-7mm, besonders bevorzugt 5-7mm. Hierdurch erfolgt eine reduzierte Fokussierung und somit wird eine sehr breite und tiefe Behandlungszone mit gewebeschonenden Druckamplituden erreicht. Dass dabei die absoluten Druckamplituden reduziert sind, ist vorteilhaft, damit es nicht zu einer Überbeanspruchung oder übermäßigen Erwärmung des Gehirngewebes und Zellschädigungen kommt.

Figuren 25a, 25b zeigen schematisch Fokussierungseffekte durch den Schädel. In Fig. 25a und 25b ist der Einfluss der Fokussierung des Schädels gezeigt. In Fig. 25a emittiert ein Applikator 3 eine leicht konvexe Stoßwelle. Durch den Schädel 244 und seine Eigenschaften (höhere Steifigkeit, höhere Wellengeschwindigkeit, konvexe Form) werden die Stoßwellen 246 zusätzlich fokussiert. Dieser Effekt sollte bei der Behandlung unbedingt berücksichtigt werden. In Fig. 25b wird gezeigt, dass mit einer divergenten abgestrahlter Stoßwelle 246` (dominanter Druckstoß) und der nachfolgenden Fokussierung des Schädels 244 eine nahezu planare Wellenausbreitung im Schädel erreicht wird. Durch die planare Wellenausbreitung im Schädel wird eine Fokussierung und hohe, schädigende Druckamplituden vermieden. Weiterhin wird durch die Berücksichtigung der Schädelkrümmung die Elektrodenposition derart optimiert, dass unter Berücksichtigung der Schädelkrümmung stets planare Wellen im Behandlungsbereich propagieren.

Figur 26 zeigt schematisch Schritte eines erfindungsgemäßen Verfahrens. Fig. 26 zeigt insbesondere die Vorgehensweise bei der Ermittlung von Behandlungsparametern. In Schritt 261 werden hochaufgelöste (idealerweise bis auf Zellebene) Patienten-Daten (CRT, MRT etc.) eingelesen (z.B. in DICOM). In Schritt 262 erfolgt die Umwandlung der Patienten-Daten für die FEM-Berechnung (z.B. mit MATLAB) sowie Zuweisung von Dichte, Elastizitätsmodul und anderen mechanischen Parametern. In Schritt 263 erfolgt die Durchführung der FEM-Berechnung (z.B. mit ANSYS) zur Simulation des Einflusses der Stoßwellen auf Zellebene (einschließlich Wiederholung mit veränderlichen Stoßwellen-Parametern oder auch veränderten Modellparametern). In Schritt 264 erfolgt die Ableitung von Behandlungsparametern für eine effektive und sichere Behandlung, also die Festlegung von Einstellparametern. Bei der Ermittlung der Behandlungsparameter kann auch eine KI unterstützen, in dem frühere Behandlungsparameter mit den ermittelten Behandlungsergebnissen bei dem aktuell zu behandelnden Patienten verglichen werden. Dann kann man z.B. bestimmte Regionen des Patientengehirns durch Erfassung dessen Feinstruktur bereits durch die KI bzw. auch festgestellter Analogie zu früheren Behandlungen die erforderlichen Behandlungsparameter ableiten bzw. in einer entsprechenden Datenbank abfragen.

## Patentansprüche

1. Vorrichtung (10; 20; 80; 90) für die extrakorporale Stoßwellentherapie, insbesondere für die Behandlung von Alzheimererkrankungen, nämlich eine elektrohydraulische Stoßwellenvorrichtung, umfassend mindestens einen Stoßwellengenerator (12) und mindestens einen Stoßwellenapplikator (3), wobei die Vorrichtung (10) einen Kondensator-Entladeschwingkreis aufweist, dessen Entladefrequenzverteilung ein Maximum besitzt, welches unter 350kHz liegt, bevorzugt unter 300 kHz, weiter bevorzugt unter 250kHz, noch weiter bevorzugt unter 200kHz, noch weiter bevorzugt unter 150kHz, noch weiter bevorzugt unter 100kHz.

2. Vorrichtung (10; 20; 80; 90) für die extrakorporale Stoßwellentherapie, insbesondere gemäß Anspruch 1, insbesondere für die Behandlung von Alzheimererkrankungen, nämlich eine elektrohydraulische Stoßwellenvorrichtung umfassend mindestens einen Stoßwellengenerator (12) und mindestens einen Stoßwellenapplikator (3), wobei die Vorrichtung (10) eine Frequenzsteuereinheit (11) umfasst, die dazu ausgelegt ist, den elektrohydraulischen Stoßwellengenerator (12) so einzustellen, dass ein Kondensator-Entladeschwingkreis der Vorrichtung (10; 20; 80; 90) eine Entladefrequenzverteilung besitzt, deren Maximum unter 350kHz liegt, bevorzugt unter 300 kHz, weiter bevorzugt unter 250kHz, noch weiter bevorzugt unter 200kHz, noch weiter bevorzugt unter 150kHz, noch weiter bevorzugt unter 100kHz liegt.

3. Vorrichtung für die extrakorporale Stoßwellentherapie gemäß Anspruch 2, wobei die Frequenzsteuereinheit (11) dazu ausgelegt ist, die Entladefrequenzverteilung über eine Veränderung der Induktivität des Entladekreises einzustellen, insbesondere über eine Festlegung der Länge eines Kabels zwischen Stoßwellengenerator (12) und Stoßwellenapplikator (3) .

4. Vorrichtung für die extrakorporale Stoßwellentherapie, insbesondere für die Behandlung von Alzheimererkrankungen, insbesondere gemäß einem der vorhergehenden Ansprüche, bevorzugt eine elektrohydraulische Stoßwellenvorrichtung, umfassend mindestens einen Stoßwellengenerator (12) und mindestens einen Stoßwellenapplikator (2), wobei die Vorrichtung (10; 20; 80; 90) eine Impulssteuereinheit (11) umfasst, die dazu ausgelegt ist, den Stoßwellengenerator (12) so einzustellen, dass eine den Stoßwellenapplikator (2) verlassende Stoßwelle eine Anstiegszeit von 6-40ns, bevorzugt 6-22ns, noch bevorzugter 9-12ns, aufweist.

5. Vorrichtung für die extrakorporale Stoßwellentherapie gemäß Anspruch 4, wobei die Impulssteuereinheit (11) dazu ausgelegt ist, die Anstiegszeit einzustellen, insbesondere über eine Veränderung der Induktivität des Entladekreises und/oder der Einstellung der Entladespannung.

6. Vorrichtung für die extrakorporale Stoßwellentherapie gemäß einem der vorhergehenden Ansprüche, wobei die Vorrichtung (10; 20; 80; 90) eine Impulsfrequenzsteuereinheit (11) umfasst, die dazu ausgelegt ist, den, bevorzugt elektrohydraulischen, Stoßwellengenerator (12) so einzustellen, dass Stoßwellen mit einer Impulsfrequenz größer gleich 15 Hz, vorzugsweise größer gleich 20 Hz, weiter bevorzugt größer gleich 40 Hz, und besonders bevorzugt im Bereich von 50Hz bis 100 Hz abgegeben werden.

7. Vorrichtung für die extrakorporale Stoßwellentherapie gemäß einem der vorhergehenden Ansprüche, umfassend mindestens einen Applikator (3) mit einem Reflektor (241) und einem Elektrodenpaar (242, 242`), und umfassend eine Fokussteuereinheit (11), die dazu ausgelegt ist, die abgegeben Stoßwellen zu fokussieren und zu defokussieren, insbesondere dazu ausgelegt ist, die Position mindestens einer Elektrode (242, 242`) im Reflektor (241) zu verschieben.

8. Vorrichtung für die extrakorporale Stoßwellentherapie gemäß einem der vorhergehenden Ansprüche, umfassend zwei oder mehr Stoßwellenapplikatoren (3) mit jeweils einem Elektrodenpaar (242, 242`), wobei jedes Elektrodenpaar (242, 242`) durch einen separaten Entladeschwingkreis gezündet wird.

9. Vorrichtung für die extrakorporale Stoßwellentherapie gemäß einem der vorhergehenden Ansprüche, umfassend eine Steueranordnung (11), wobei die Steueranordnung (11) dazu ausgelegt ist, einen Betriebsmodus zur Behandlung von Alzheimererkrankungen einzunehmen und in diesem vorbestimmte Einstellparameter für die Frequenzsteuereinheit, die Impulssteuereinheit und/oder der Impulsfrequenzsteuereinheit festlegt.

10. Vorrichtung für die extrakorporale Stoßwellentherapie gemäß einem der vorhergehenden Ansprüche, wobei es sich bei der Vorrichtung für die extrakorporale Stoßwellentherapie um eine elektrohydraulische Stoßwellenvorrichtung mit Selbstzündung handelt.

11. Vorrichtung für die extrakorporale Stoßwellentherapie gemäß einem der vorhergehenden Ansprüche, wobei es sich bei der Vorrichtung für die extrakorporale Stoßwellentherapie um eine elektrohydraulische Stoßwellenvorrichtung handelt, wobei
- der Entladeschwingkreis der Vorrichtung einen Kondensator aufweist mit einer Kapazität von 10-800 nF, insbesondere 50-400 nF, weiter insbesondere 100-300 nF, weiter insbesondere 150 - 250 nF,
und/oder
- der Entladeschwingkreis der Vorrichtung einen Kondensator aufweist mit einer Ladespannung von 1kV und 20 kV, insbesondere zwischen 1kV und 10 kV, weiter insbesondere zwischen 1kV und 7.5kV
und/oder
- der Entladeschwingkreis der Vorrichtung einen Kondensator aufweist und die in dem Kondensator gespeicherte Energie des Stoßwellengerätes bei 0.5J bis 25J liegt, insbesondere bei 0.5J bis 5J.

12. System zur Behandlung von Alzheimererkrankungen umfassend eine Vorrichtung (10; 20; 80; 90) für die extrakorporale Stoßwellentherapie gemäß einem der vorhergehenden Ansprüche, und
umfassend eine Prozessoreinheit, die dazu ausgelegt ist,
- Diagnosedaten von einem Datenspeicher oder einer Diagnosevorrichtung, insbesondere von einer Bildgebungsvorrichtung oder einer Vorrichtung zur Analyse von Amyloid AD Blutwerten, zu empfangen,
- Einstellparameter für die Vorrichtung (10; 20; 80; 90) für die extrakorporale Stoßwellentherapie in Abhängigkeit der Diagnosedaten zu bestimmen,
- die Einstellparameter an die Vorrichtung (10; 20; 80; 90) für die extrakorporale Stoßwellentherapie abzugeben.

13. System zur Behandlung von Alzheimererkrankungen gemäß Anspruch 12, wobei die Einstellparameter gewählt sind aus der Gruppe
- Anzahl, Position und/oder Ausrichtung der Stoßwellenapplikatoren,
- Entladefrequenz des Entladeschwingkreises
- Anstiegszeit der Stoßwelle
- Leistungsstufe bzw. Ladespannung des Kondensators
- Fokussierungsgrad,
- Impulsfrequenz (bzw. Impulsrate)
- Anzahl der zu applizierenden Stoßwellen
- Sequenzen der einzelnen Stoßwellen
- Wiederholrate von Impulssequenzen.

14. System zur Behandlung von Alzheimererkrankungen gemäß Anspruch 12 oder 13, wobei die Diagnosevorrichtung eine Bildgebungsvorrichtung für 3d-Darstellungen des Kopfes ist und die Prozessoreinheit insbesondere dazu ausgelegt ist, Propagationen von Stoßwellen in einem Kopf aufgrund der Bilddaten zu simulieren und in Abhängigkeit der Simulation optimierte Einstellparameter zu bestimmen.

15. System zur Behandlung von Alzheimererkrankungen gemäß einem der Ansprüche 12-14, wobei das System eine Ultraschallvorrichtung und eine Kombinations-Steueranordnung umfasst, die insbesondere dazu ausgelegt ist, Ultraschall und Stoßwellen zeitnah in denselben Behandlungsbereich zu applizieren.

16. System zur Behandlung von Alzheimererkrankungen gemäß einem der Ansprüche 12-15, wobei das System eine Medikamentenabgabevorrichtung umfasst und die Prozessoreinheit insbesondere dazu ausgelegt ist,
- Abgabeparameter für die Medikamentenabgabevorrichtung in Abhängigkeit der Diagnosedaten zu bestimmen und
- die Abgabeparameter an die Medikamentenabgabevorrichtung abzugeben.

17. System zur Behandlung von Alzheimererkrankungen gemäß einem der Ansprüche 12 bis 16, wobei die Vorrichtung eine Temperiervorrichtung (157) für den Kopf des Patienten umfasst und die Prozessoreinheit insbesondere dazu ausgelegt ist,
- Temperierparameter für die Temperiervorrichtung (157) in Abhängigkeit der Diagnosedaten zu bestimmen und
- die Temperierparameter an die Temperiervorrichtung (157) abzugeben.

18. Verfahren zum Bereitstellen von Einstellparametern für eine Behandlung von Alzheimererkrankungen mit einem System (100) gemäß einem der Ansprüche 12-17, umfassend die Schritte
- Bereitstellen von Diagnosedaten von einem Datenspeicher oder einer Diagnosevorrichtung, insbesondere von einer Bildgebungsvorrichtung oder einer Vorrichtung zur Analyse von Blutwerten hinsichtlich Amyloid,
- Bestimmen von Einstellparameter für die Vorrichtung (10; 20; 80; 90) für die extrakorporale Stoßwellentherapie in Abhängigkeit der Diagnosedaten,
- insbesondere Bestimmen von Einstellparameter für eine Ultraschallvorrichtung, Bestimmen von Abgabeparametern für eine Medikamentenabgabevorrichtung und/oder Bestimmen von Temperierparametern für eine Temperiervorrichtung (157) .

19. Computerprogramm mit Programmcode zur Durchführung der Schritte des Verfahrens nach Anspruch 18, wenn das Programm auf einer Prozessoreinheit eines Systems (100) gemäß einem der Ansprüche 12-17 ausgeführt wird.

20. Computerprogrammprodukt, das direkt in einen internen Speicher eines Digitalcomputers geladen werden kann und das Softwarecodeabschnitte umfasst, die die Verfahrensschritte des Verfahrens nach Anspruch 18 ausführen, wenn das Programm auf dem Digitalcomputer eines Systems (100) gemäß einem der Ansprüche 12-17 ausgeführt wird.
